(19) Europäisches Patentamt
European Patent Office
Office européen des brevets

(11) EP 2 818 557 A1

(12) **EUROPEAN PATENT APPLICATION**

(43) Date of publication:
**31.12.2014 Bulletin 2015/01**

(21) Application number: **13003212.1**

(22) Date of filing: **24.06.2013**

(51) Int Cl.:
*C12P 19/06* (2006.01)
*C12N 9/18* (2006.01)
*C08B 37/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Westfälische Wilhelms-Universität Münster 48149 Münster (DE)**
- **Wageningen Universiteit 6708 PB Wageningen (NL)**

(72) Inventors:
- **Kool, Marjin 3523 KH Utrecht (NL)**
- **Schols, Henk Dr., 6707 HG Wageningen (NL)**
- **Moerschbacher, Bruno Prof. Dr., 48161 Münster (DE)**
- **Wagenknecht, Martin Dr., 48155 Münster (DE)**

(74) Representative: **Lahrtz, Fritz Isenbruck Bösl Hörschler LLP Patentanwälte Prinzregentenstrasse 68 81675 München (DE)**

(54) **Process for the site-specific deacetylation of xanthan employing esterases**

(57) The present invention relates to a method for producing xanthan with an altered acetylation pattern, wherein an enzyme site-specifically cleaving acetyl residues off mannose moieties of acetylated xanthan is contacted with acetylated xanthan. Furthermore, the present invention refers to the xanthan with the characteristics obtainable from such method and to the use thereof. Moreover, the present invention also refers to the use of enzymes site-specifically cleaving off the acetyl residues of mannose moieties of acetylated xanthan.

Fig. 4

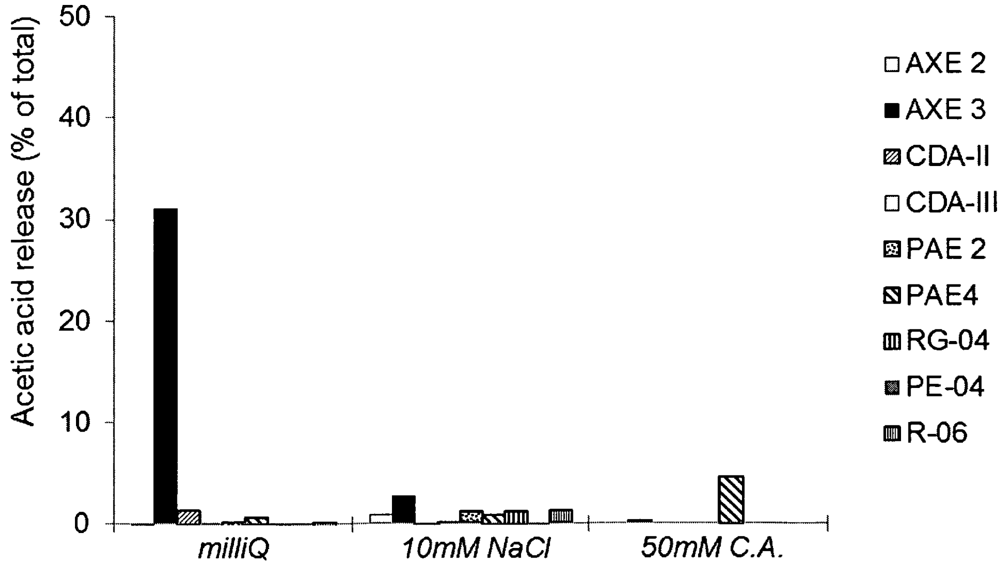


EP 2 818 557 A1

<110> Westfälische Wilhelms-Universität Münster
Wageningen Universiteit

<120> Means and Methods for Producing Deacetylated Xanthan

<130> P68600EP

<160> 2

<170> PatentIn version 3.5

<210> 1
<211> 313
<212> PRT
<213> Chrysosporium lucknowense

<400> 1

```
Met Lys Leu Leu Gly Lys Leu Ser Ala Ala Leu Ala Leu Ala Gly Ser
1               5                   10                  15

Arg Leu Ala Ala Ala His Pro Val Phe Asp Glu Leu Met Arg Pro Thr
            20                  25                  30

Ala Pro Leu Val Arg Pro Arg Ala Ala Leu Gln Gln Val Thr Asn Phe
        35                  40                  45

Gly Ser Asn Pro Ser Asn Thr Lys Met Phe Ile Tyr Val Pro Asp Lys
    50                  55                  60

Leu Ala Pro Asn Pro Pro Ile Ile Val Ala Ile His Tyr Cys Thr Gly
65                  70                  75                  80

Thr Ala Gln Ala Tyr Tyr Ser Gly Ser Pro Tyr Ala Arg Leu Ala Asp
                85                  90                  95

Gln Lys Gly Phe Ile Val Ile Tyr Pro Glu Ser Pro Tyr Ser Gly Thr
            100                 105                 110

Cys Trp Asp Val Ser Ser Arg Ala Ala Leu Thr His Asn Gly Gly Gly
        115                 120                 125

Asp Ser Asn Ser Ile Ala Asn Met Val Thr Tyr Thr Leu Glu Lys Tyr
    130                 135                 140

Asn Gly Asp Ala Ser Lys Val Phe Val Thr Gly Ser Ser Ser Gly Ala
145                 150                 155                 160

Met Met Thr Asn Val Met Ala Ala Ala Tyr Pro Glu Leu Phe Ala Ala
                165                 170                 175

Gly Ile Ala Tyr Ser Gly Val Pro Ala Gly Cys Phe Tyr Ser Gln Ser
            180                 185                 190

Gly Gly Thr Asn Ala Arg Asn Ser Ser Cys Ala Asn Gly Gln Ile Asn
        195                 200                 205
```

Ser Thr Pro Gln Val Trp Ala Lys Met Val Phe Asp Met Tyr Pro Glu
210 215 220

Tyr Asp Gly Pro Arg Pro Lys Met Gln Ile Tyr His Gly Ser Ala Asp
225 230 235 240

Gly Thr Leu Arg Pro Ser Asn Tyr Asn Glu Thr Ile Lys Gln Trp Cys
245 250 255

Gly Val Phe Gly Phe Asp Tyr Thr Arg Pro Asp Thr Thr Gln Ala Asn
260 265 270

Ser Pro Gln Ala Gly Tyr Thr Thr Tyr Thr Trp Gly Glu Gln Gln Leu
275 280 285

Val Gly Ile Tyr Ala Gln Gly Val Gly His Thr Val Pro Ile Arg Gly
290 295 300

Ser Asp Asp Met Ala Phe Phe Gly Leu
305 310

<210> 2
<211> 217
<212> PRT
<213> Bacillus subtilis

<400> 2

Met Ala Asn His Ile Tyr Leu Ala Gly Asp Ser Thr Val Gln Thr Tyr
1 5 10 15

Gly Asp Ser Thr Asn Gln Gly Gly Trp Gly Gln Phe Leu Gly Ser His
20 25 30

Leu Pro Glu His Ile Gln Val Ile Asn Arg Ala Ile Gly Gly Arg Ser
35 40 45

Ser Lys Thr Phe Val Glu Glu Gly Arg Leu Gln Ala Ile Leu Asp Val
50 55 60

Ile Glu Pro Asp Asp Trp Leu Phe Val Gln Met Gly His Asn Asp Ala
65 70 75 80

Ser Lys Asn Lys Pro Glu Arg Tyr Thr Glu Pro Tyr Thr Thr Tyr Lys
85 90 95

Gln Tyr Leu Lys Gln Tyr Ile Ala Gly Ala Arg Glu Lys Gly Ala His
100 105 110

Pro Leu Leu Ile Thr Pro Val Ala Arg Phe His Tyr Glu Asn Gly Val
115 120 125

Phe Leu Asn Asp Phe Pro Asp Tyr Cys Ile Ala Met Lys Gln Thr Ala

```
Ala Glu Glu Asn Val Gln Leu Ile Asp Leu Met Glu Lys Ser Leu Ala
145                 150                 155                 160

Phe Phe Thr Glu Lys Gly Glu Glu Lys Val Tyr Thr Tyr Phe Met Ile
                165                 170                 175

Ser Glu Gly Ile Asn Asp Tyr Thr His Phe Thr Lys Lys Gly Ala Asn
            180                 185                 190

Glu Met Ala Lys Leu Val Ala Lys Gly Ile Lys Glu Leu Gly Leu Pro
        195                 200                 205

Leu Thr Glu Ser Ile Ile Lys Glu Arg
    210                 215
```

## Description

The present invention relates to a method for producing xanthan with an altered acetylation pattern, wherein an enzyme site-specifically cleaving acetyl residues off mannose moieties of acetylated xanthan is contacted with acetylated xanthan. Furthermore, the present invention refers to the xanthan with the characteristics obtainable from such method and to the use thereof. Moreover, the present invention also refers to the use of enzymes site-specifically cleaving off the acetyl residues of mannose moieties of acetylated xanthan.

Xanthan is a well-known polysaccharide that has a cellulose-like β-1,4-glucan backbone structure and further comprises (3→1) linked α-D-mannose-(2→1)-β)-D-glucuronic acid-(4→1)β-D-mannose side chains that are typically located on every second glucose unit (Jansson, Kenne and Lindberg, 1975). Xanthan can be obtained from natural sources. Exemplarily, *Xanthomonas* bacteriae are used in the production of xanthan, in particular those of the species *Xanthomonas campestris.*

When dissolved in water, xanthan solutions exhibit pseudo-plastic properties, even at low xanthan concentrations. These properties are highly stable over a wide pH and temperature range, making xanthan suitable for many food applications. Today, xanthan is already used in food industry as food stabilizer and rheological modifier.

However, its applicability in many fields is still severely hampered by some considerably drawbacks with respect of its rheological properties.

It has been considered that the presence of too many acetyl residues conjugated to the xanthan in some positions may influence the desired rheological properties negatively. Naturally occurring xanthan typically bears one or more acetyl residues conjugated to one or both of the mannose moieties. In fact, recent studies have revealed that approximately 85 to 90 % of all inner mannose units are acetylated at the O-6 position and about 50 to 70 % of all terminal mannose units are substituted with a pyruvic acid acetal. Additionally, about 5 to 25 % of all terminal mannose units can be substituted with an acetyl group (Stankowski, Mueller and Zeller, 1993; Kool et al., 2013b).

The acetyl and pyruvate groups of xanthan are known to have a great influence on the viscosity of xanthan solutions and its stability towards the addition of salts and changes in acidity (Morrison, Clark, Talashek and Yuan, 2004; Shatwell, Sutherland, Dea and Ross-Murphy, 1990). Lowering the degree of acetylation results in xanthan solutions with better and more stable physical properties and in improved interactions with galactomannans (Morrison, Clark, Talashek and Yuan, 2004; Shatwell, Sutherland, Ross-Murphy and Dea, 1990).

It is known that the chemical removal of acetyl groups may improve the properties of the xanthan and its interactions with galactomannans. To date, acetyl groups are removed using an acidic or, in particular, alkali treatment (Bradshaw, Nisbet, Kerr and Sutherland, 1983; Pinto, Furlan and Vendruscolo, 2011; Tako and Nakamura, 1984).

However, such method based on synthetic chemical means is a very rough procedure and bears several severe drawbacks. First, the removal of acetyl residues is entirely unspecific and random and cannot be controlled properly. The rheological properties of the xanthan that will be obtained from such a method can previously not even be roughly assessed. Secondly, in the chemical reaction toxic and unwanted agents, such as exemplarily alkaline agents, are used that have to be thoroughly removed from the obtained xanthan after the removal of acetyl residues is completed. This requires additional laborious method steps. Thirdly, removal of acetyl residues by synthetical chemical means often also results in a partly degradation of the xanthan's backbone what is entirely unwanted.

An alternative method to control the acetyl levels in xanthan is the use of specific *Xanthomonas* strains and/or fermentation conditions for the xanthan production (Flores Candia and Deckwer, 1999; Hassler and Doherty, 1990; Peters, Suh, Schumpe and Deckwer, 1993).

Altering the production conditions of xanthan, however, influences the pyruvate levels in the produced xanthan. As the pyruvate groups are also of great importance for xanthans functionality (Sandford, Pittsley, Knutson, Cadmus, Watson and Jeanes, 1977; Shatwell, Sutherland, Ross-Murphy and Dea, 1990; Smith, Symes, Lawson and Morris, 1984), these techniques are also not applicable to modify only the acetyl content of xanthan.

Therefore, there is still a need for a method for mild and selective removal of acetyl groups from the xanthan side chains in a controlled way. Furthermore, there is also a need for xanthans with improved technical properties and broadened applicability.

Surprisingly, as laid out in detail below, a method for mild and selective removal of acetyl groups from the xanthan side chains in a controlled way was found that entirely does not affect the xanthan backbone. Additionally, xanthan with an altered acetylation pattern and improved technical properties was obtained.

In a first aspect, the present invention therefore relates to a method for producing xanthan with an altered acetylation pattern, wherein an enzyme site-specifically cleaving acetyl residues off mannose moieties of acetylated xanthan is contacted with acetylated xanthan.

As used herein, the terms "method", "process", "procedure" and "technique" may be understood exchangeably and in their broadest sense.

"Xanthan" in the context of the present invention may be understood in the broadest sense as any xanthan known in the art. Preferably, xanthan is a polysaccharide comprising a β-1,4-glucan backbone and further comprises on approximately every second glucose unit a (3→1) linked α-D-mannose-(2→1)-β-D-glucuronic acid-(4→1)-β-D-mannose side chain. Naturally occurring xanthan typically bears one or more acetyl residues conjugated to one or both of the mannose moieties. An exemplary generic structure of xanthan is depicted in Figure 1A. Further examples of xanthan repeating units are depicted in Figure 1B.

The xanthan in the context of the present invention is acetylated xanthan. Herein, the term "acetylated xanthan" may be understood in the broadest sense as any xanthan molecule comprising at least one acetyl residue, preferably at least two, more preferably at least five, even more preferably at least ten, even more preferably at least 50 acetyl residues, even more preferably at least 100 acetyl residues, even more preferably at least 1000 acetyl residues or even more residues. More preferably, acetylated xanthan comprises at least one acetyl residue, preferably at least two, more preferably at least five, even more preferably at least ten, even more preferably at least 50 acetyl residues at mannose moieties. The inner mannose groups are those attached to the backbone of xanthan, whereas the outer are those terminal at the side chains. Highly preferably, at least 10 %, at least 20 %, at least 30 %, at least 40 %, at least 50 %, at least 60 %, at least 70 %, at least 80 % or at least 90% of all inner mannose units are acetylated at the O-6 position and/or at least 10 %, at least 20 %, at least 30 %, at least 40 %, at least 50 %, at least 60 %, at least 70 %, at least 80 % or at least 90% of all terminal mannose units may be substituted with a pyruvic acid acetal. In particular, herein, at least 10 %, at least 20 %, at least 30 %, at least 40 % or at least 50 % of all terminal mannose units may be substituted with a pyruvic acid acetal.

The term "altered acetylation pattern" as used herein refers to removing and/or adding of acetyl residues on the xanthan. This may overall result in xanthan with a reduced number of acetyl residues, with an essentially equal number of acetyl residues or in an increased number of acetyl residues.

Preferably, the number of acetyl residues in the site-specific mannose moieties of the xanthan is reduced, in particular at least 1.1-fold, at least 1.5-fold, at least 2-fold, at least 5-fold, at least 10-fold or at least 20-fold.

Alternatively or additionally, incubating the mixture may or may not comprise conjugating one or more acetyl residue(s) to xanthan at specific positions in the xanthan.

Herein, the terms "site-specifically cleaving off" may be understood in the broadest sense as the selectively preferred removal of acetyl residue(s) at particular positions. Preferably, at the specific positions, the yield of removal of acetyl residue(s) is at least 1.1-fold, at least 1.5-fold, at least 2-fold, at least 5-fold, at least 10-fold or at least 20-fold more effective that at other positions in the acetylated xanthan.

The term "enzyme" as used herein may be understood in the broadest sense as any macromolecule, i.e., a molecule of a molecular weight (MW) of more than 500 Da, than catalyzes one or more chemical reaction(s). In the context of the present invention, an enzyme catalyzes the cleavage of the ester bond of acetyl residues off mannose moieties of the xanthan. In this context, acetylated xanthan may serve as the substrate (also: the educt) and xanthan with an altered acetylation pattern may be the product. The person skilled in the art will immediately notice that, depending on the concentration ratios between substrates and educts, the kinetic of the reaction may vary and that the reaction may even be reversed, wherein upon reversal of the reaction the designations "substrate" and "educt" may also be reversed. Preferably, the enzyme is a polypeptide.

As used throughout the present invention, the terms "polypeptide", "peptide" and "protein" may be understood interchangeably in the broadest sense as any molecule comprising a strand of at least four, preferably of at least ten, more preferably of at least 20, even more preferably of at least 50 consecutive amino acids.

A polypeptide in the sense of the present invention may also include one or more posttranslational modification(s). A plurality of posttranslational modifications are well-known in the art and may comprise but may not be limited to lipidation, phosphorylation, sulfatation, glycosylation, truncation, cyclization of several amino acid moieties, cyclization of the polypeptide strand, oxidation, reduction, decarboxylation, acetylation, amidation, deamidation, disulfide bond formation, pyroglutamate formation, amino acid addition, cofactor addition (e.g., biotinylation, heme addition) and complexation of metal ions, non-metal ions, peptides or small molecules and addition of iron-sulphide clusters. Evidently, co-factors, such as the binding of ATP, ADP, NAD+, NADH+H+, NADP+, NADPH+H+, metal ions, anions, lipids, etc. may be bound to the polypeptide, irrespective on the biological influence of these co-factors.

Optionally, a polypeptide in the sense of the present invention may also be conjugated or bound to one or more other molecule(s) that may preferably be one or more of the group consisting of but not limited to fusion proteins (e.g., fluorescent proteins (e.g., cyan fluorescent protein (CFP), green fluorescent protein (GFP) or yellow fluorescent protein (YFP), red fluorescent protein (RFP), mCherry, etc.)), one or more lipids or lipidoids, one or more small molecule dyes (e.g., fluorescent dyes (e.g., a Cy dye (e.g., Cy3, Cy5, Cy5.5, Cy 7), an Alexa dye (e.g., Alexa Fluor 488, Alexa Fluor 546, Alexa Fluor 647, Alexa Fluor 680, Alexa Fluor 750), a Visen dye (e.g. VivoTag680, VivoTag750), an S dye (e.g., S0387), a DyLight fluorophore (e.g., DyLight 750, DyLight 800), an IRDye (e.g., IRDye 680, IRDye 800), a fluorescein dye (e.g., fluorescein, carboxyfluorescein, fluorescein isothiocyanate (FITC)), a rhodamine dye (e.g., rhodamine, tetramethylrhodamine (TAMRA)) or a HOECHST dye) or UV/Vis dyes (e.g., a p-nitrophenyl moiety, Coomassie Brilliant Blue G-250), one or more quantum dots, one or more binding moieties (e.g., biotin, methotrexate, glycocorticoids or moieties comprising, e.g., one or more active esters, isothiocyanates, maleimides, N-hydroxysuccinimidyl esters, glutaraldehyde derivatives, carbodiimide derivatives or combinations thereof), one or more insoluble and/or soluble polymers (e.g., polyethylene glycol (PEG), hydroxypropyl methacrylate (HPMA), polyethylene imine (PEI)), one or more antibodies or derivatives thereof (e.g., Fab fragments, single chain antibodies, diabodies, triabodies, flexibodies, tandabs), one or more peptides (e.g., cell-penetrating peptides (CPPs), protein transduction domains (PTDs), signal transduction peptides, etc.), one or more spin labels, one or more enzyme labels (e.g., penicillinase, horseradish peroxidase, alkaline phosphatase), micro- or nanobeads (e.g., functionalized silica beads, polysaccharide-based beads), polymersomes and/or liposomes.

Further, the polypeptide may optionally be radioactively labeled by, e.g., one or more of the group consisting of but nut limited to $^{3}H$, $^{32}P$, $^{35}S$, $^{14}C$, $^{99}Tc$ or lanthonoids (e.g., $^{64}Gd$). Further, the polypeptide may optionally be spin-labeled by, e.g., $^{2}H$ and/or $^{13}C$.

Contacting in the sense of the present invention may be contacting by any means. Preferably contacting is performed by dissolving or emulsifying the acetylated xanthan in the same batch as the polypeptide or derivative thereof of the present invention, wherein the batch further comprises a liquid solvent. Highly preferably, the liquid solvent is an aqueous solvent, in particular an aqueous buffer. Such aqueous buffer preferably maintains the enzymatic activity of the polypeptide or derivative thereof of the present invention.

Preferably, in the method of the present invention, in general, the xanthan is not obtained from genetically modified organisms (GMOs), in particularly no organisms are used in which the xanthan acetylation has been genetically influenced by any technical means such as by amending the acetylation pattern by knocking out or down one or more genes encoding for enzymes responsible for acetylation and/or deacetylation.

When no xanthan obtained from GMOs is used, this may have several advantages. Typically, in GMOs, one or more genes involved in acetylating mannose are completely deleted what may on the one hand result in deacetylated xanthan, but does not allow intermediate levels of acetylation at either position on mannose, merely complete absence or presence of acetyl residues in possible. In contrast, the present invention has the significant improvement in specific and optionally partly removal of acetyl residues. Furthermore, xanthan with altered acetylation pattern produced in the absence of GMOs may have higher consumer acceptance.

According to the present invention, the enzyme may cleave acetyl residues off any mannose.

In a preferred embodiment, the enzyme site-specifically cleaves acetyl residues off the inner mannose and/or off the outer mannose moieties of acetylated xanthan.

Therefore, the enzyme may site-specifically cleave acetyl residues off the inner mannose. An example for such enzyme is AXE3 which is an enzyme that selectively cleaves acetyl residues off the inner mannose. Alternatively, the enzyme may site-specifically cleave acetyl residues off the outer mannose. An example for such enzyme is YesY which is an enzyme that selectively cleaves acetyl residues off the outer mannose. Alternatively, the enzyme may site-specifically simultaneously cleave acetyl residues off the inner and outer mannose, therefore may be bispecific for acetyl residues at inner and outer mannose moieties.

Therefore, the enzyme may be any enzyme that site-specifically cleaves acetyl residues off the inner and/or outer mannose, therefore any enzyme bearing a site-specific mannose deacetylating activity.

As used herein, the term "deacetylating" may be understood in the broadest sense as removing acetyl residues from the xanthan molecule. Herein, the xanthan molecule may also be designated as "bare xanthan molecule". The person skilled in the art will immediately notice that removing acetyl residues refers to the cleavage of the C-O bond in the ester group between the acetyl residues and the xanthan backbone typically resulting in an increased number of hydroxyl groups on the xanthan. Removing acetyl residues from the xanthan does not necessary mean that the acetate anions liberated thereby are removed from the sample. As used herein, the terms "acetyl residue", "acetyl group" and "acetyl residue" may be understood interchangeably.

The enzyme may enzyme site-specifically cleave acetyl residues off the inner and/or the outer mannose moieties of acetylated xanthan.

In a preferred embodiment, the enzyme site-specifically cleaves acetyl residues off the inner mannose moieties of acetylated xanthan.

In this context, an enzyme site-specifically cleaving acetyl residues off the inner mannose may be any enzyme having this feature known in the art.

In a more preferred embodiment, the enzyme cleaving acetyl residues off the inner mannose is an acetyl xylan esterase (AXE), preferably a fungal AXE, more preferably an AXE from *Chrysosporium lucknowense,* in particular a polypeptide of at least 80% homology to SEQ ID NO: 1 or a functional derivative thereof having at least 10 % of its xanthan-deacetylating activity.

*Myceliophtora thermophila* has previously also been designated as *Chrysosporium lucknowense.* Therefore these designations may be understood exchangeably throughout the present invention.

As used herein, *Chrysosporium lucknowense* is preferably, *Chrysosporium lucknowense* C1 (corresponding to *Myceliopthora thermophila* C1).

Throughout the present invention SEQ ID NO: 1, refers to the following amino acid sequence:

MKLLGKLSAALALAGSRLAAAHPVFDELMRPTAPLVRPRAALQQVTNFGSNPSN
TKMFIYVPDKLAPNPPIIVAIHYCTGTAQAYYSGSPYARLADQKGFIVIYPESPYSG
TCWDVSSRAALTHNGGGDSNSIANMVTYTLEKYNGDASKVFVTGSSSGAMMTN
VMAAAYPELFAAGIAYSGVPAGCFYSQSGGTNARNSSCANGQINSTPQVWAKM
VFDMYPEYDGPRPKMQIYHGSADGTLRPSNYNETIKQWCGVFGFDYTRPDTTQ
ANSPQAGYTTYTWGEQQLVGIYAQGVGHTVPIRGSDDMAFFGL

Herein, the first 21 amino acids may be considered as a signal peptide. Therefore, the mature polypeptide may optionally also start at the amino acid moiety in position 22 of the above sequence.

Preferably, the polypeptide of the present invention comprises an amino acid sequence of at least 80% homology to SEQ ID NO: 1, more preferably of at least 85% homology to SEQ ID NO: 1, even more preferably of at least 90% homology to SEQ ID NO: 1, even more preferably of at least 95% homology to SEQ ID NO: 1 and in particular an amino acid sequence of SEQ ID NO: 1.

Highly preferably, the polypeptide of the present invention consists of an amino acid sequence of at least 80% homology to SEQ ID NO: 1, more preferably of at least 85% homology to SEQ ID NO: 1, even more preferably of at least 90% homology to SEQ ID NO: 1, even more preferably of at least 95% homology to SEQ ID NO: 1 and in particular of an amino acid sequence of SEQ ID NO: 1.

A polypeptide comprising SEQ ID NO: 1 or a derivative thereof preferably cleaves off acetyl residues located at the inner mannose.

In an alternative preferred embodiment, the enzyme site-specifically cleaves acetyl residues off the outer mannose moieties of acetylated xanthan.

Herein, an enzyme site-specifically cleaving acetyl residues off the outer mannose may be any enzyme having this feature known in the art.

In a more preferred embodiment, the enzyme cleaving acetyl residues off the outer mannose is a pectin acetylesterase, preferably a rhamnogalacturonan acetylesterase, more preferably a rhamnogalacturonan acetylesterase of the *YES* cluster of *Bacillus subtilis,* in particular a polypeptide of at least 80% homology to SEQ ID NO: 2 or a functional derivative thereof having at least 10 % of its xanthan-deacetylating activity.

As used herein, a rhamnogalacturonan acetylesterase may also be designated as a pectin acetyl esterase or homogalacturonan acetyl esterase as these enzymes may deacetylate the homogalacturonan regions of pectin.

*Bacillus subtilis* is preferably but not necessarily *Bacillus subtilis* of commercially available strain 168.

Throughout the present invention, SEQ ID NO: 2 refers to the following amino acid sequence:

MANHIYLAGDSTVQTYGDSTNQGGWGQFLGSHLPEHIQVINRAIGGRSSKTFVE
EGRLQAILDVIEPDDWLFVQMGHNDASKNKPERYTEPYTTYKQYLKQYIAGARE

KGAHPLLITPVARFHYENGVFLNDFPDYCIAMKQTAAEENVQLIDLMEKSLAFFTE
KGEEKVYTYFMISEGINDYTHFTKKGANEMAKLVAKGIKELGLPLTESIIKER

Preferably, the polypeptide of the present invention comprises an amino acid sequence of at least 80% homology to SEQ ID NO: 2, more preferably of at least 85% homology to SEQ ID NO: 2, even more preferably of at least 90% homology to SEQ ID NO: 2, even more preferably of at least 95% homology to SEQ ID NO: 2 and in particular an amino acid sequence of SEQ ID NO: 2.

Highly preferably, the polypeptide of the present invention consists of an amino acid sequence of at least 80% homology to SEQ ID NO: 2, more preferably of at least 85% homology to SEQ ID NO: 2, even more preferably of at least 90% homology to SEQ ID NO: 2, even more preferably of at least 95% homology to SEQ ID NO: 2 and in particular of an amino acid sequence of SEQ ID NO: 2.

A polypeptide comprising SEQ ID NO: 2 or a derivative thereof preferably cleaves off acetyl residues located at the outer mannose.

In the context of the present invention, the term "homology" may be understood as sequence homology and/or three dimensional (3D) structural homology. Preferably, homology is sequence homology.

The enzymes in the context of the present invention maybe obtained from any means known in the art. Exemplarily, the enzymes may be obtained from the organism these sequences originate from or may be heterologically expressed in another organism such as, e.g., a bacterium (e.g., *E. coli*), a fungal cell or an animal cell (e.g., an insect or a mammalian cell).

As used in the context of the present invention, the term "functional derivative" may be understood in the broadest sense as any polypeptide that is structurally deduced from a polypeptide of SEQ ID NO: 1 and that has at least 10 %, preferably at least 20 %, more preferably at least 30 %, even more preferably at least 40 %, even more preferably at least 50 %, even more preferably at least 60 %, even more preferably at least 70 %, even more preferably at least 80 %, even more preferably at least 90 % or even 100 % or more of the xanthan-deacetylating activity of a polypeptide of a polypeptide comprising an amino acid sequence of at least 80% homology to SEQ ID NO: 1 and SEQ ID NO: 2, respectively, in particular a polypeptide of SEQ ID NO: 1 and SEQ ID NO: 2, respectively.

As used herein, "structurally deduced" means that its sequence is structurally related, i.e., that the functional derivative comprises a sequence of at least 20, preferably of at least 30, more preferably of at least 40, even more preferably of at least 50 consecutive amino acids, even more preferably of at least 75 consecutive and even more preferably of at least 100 consecutive amino acids with a sequence homology of at least 80% to the most homologous sequence interception of a SEQ ID NO: 1 and SEQ ID NO: 2, respectively.

It will be understood that a functional derivative does not necessarily have to be directly obtained from a polypeptide comprising an amino acid sequence of at least 80% homology. It may also be obtained by any biotechnological means or even by chemical peptide synthesis.

Exemplarily, a functional derivative may be a mutated form of a polypeptide comprising an amino acid sequence of at least 80% homology to SEQ ID NO: 1 and SEQ ID NO: 2, respectively. A mutated form may be a polypeptide in which one or more amino acid residues have been inserted, removed and/or exchanged. Such mutated form of the polypeptide may refer to but may not be limited to the polypeptide encoded by an altered DNA or RNA sequence, a splice variant of the polypeptide or an analogue polypeptide from another species (e.g., an animal, a viral or a bacterial polypeptide), most preferably a bacterial polypeptide. An altered DNA or RNA sequence encoding for the functional derivative may occur naturally throughout the population tested or may be of artificial origin. Alternatively, the functional derivative of the polypeptide may be of synthetical origin, i.e. chemically modified and/or synthesized.

The enzymes as described above, a composition for amending the acetylation pattern of xanthan in the sense of the present invention may comprise:

(a) an enzyme site-specifically cleaving acetyl residues off the inner mannose moieties, in particular a polypeptide sequence of at least 80% homology to SEQ ID NO: 1 or a functional derivative thereof having at least 10 % of its xanthan-deacetylating activity; and/or
(b) an enzyme site-specifically cleaving acetyl residues off the outer mannose moieties, in particular a polypeptide sequence of at least 80% homology to SEQ ID NO: 2 or a functional derivative thereof having at least 10 % of its xanthan-deacetylating activity,

Wherein (a) and (b) may be administered in combination with another or an enzyme of (a) may be administered in the absence of an enzyme of (b) or an enzyme of (b) may be administered in the absence of an enzyme of (a).

In a particularly preferred embodiment, an enzyme site-specifically cleaving acetyl residues off the inner mannose moieties of acetylated xanthan and an enzyme site-specifically cleaving acetyl residues off the outer mannose moieties of said acetylated xanthan are contacted with acetylated xanthan.

The person skilled in the art will immediately notice that such composition typically will further comprise a solvent, in particular an aqueous solvent such as an aqueous buffer. Optionally, the composition may further comprise one or more cofactors of the enzymes. Optionally, the composition may further comprise one or more further enzymes, in particular those amending the acetylation pattern of xanthan and/or digesting or producing xanthan. Preferably, the composition is essentially free of cells, more preferably essentially free of cells of genetically modified organisms (GMOs), in particular those GMOs in which the xanthan acetylation has been genetically influenced by any technical means such as by amending the acetylation pattern by knocking out or down one or more genes encoding for enzymes responsible for acetylation and/or deacetylation. Preferably, the composition is essentially free of toxic agents (e.g., organic solvents (e.g. chloroform, toluol, phenol, acetone, acetonitrile, etc.), heavy metals, etc.) and other unwanted agents (e.g., ethyl alcohol, glycerol, dimethyl sulfoxide (DMSO), etc.).

As used herein, the method according to the present invention may be conducted by any means known in the art and by using any methodic steps suitable for this purpose.

In a preferred embodiment, the method according to the present invention comprises the steps of:

(i) providing acetylated xanthan,
(ii) contacting said acetylated xanthan with one or more enzyme(s), in particular wherein at least one enzyme is an enzyme as described above,
(iii) incubating the mixture obtained from step (ii) at conditions allowing said enzyme(s) to alter the acetylation pattern of the acetylated xanthan,

(iv) recovering the xanthan with altered acetylation pattern obtained from step (iii).

Providing acetylated xanthan may be performed by any means known in the art. Therefore, the xanthan may be obtained from any source, such as, e.g, obtained from a commercial supplier or obtained from fungal cells and/or bacteria.

Recovering of the xanthan with altered acetylation pattern may be performed by any means known in the art. Exemplarily, the xanthan with altered acetylation pattern obtained from the method of the present invention may be obtained by chromatographic means (e.g., size exclusion chromatography, fast protein liquid chromatography (FPLC), high performance liquid chromatography (HPLC) and/or ultra high performance liquid chromatography (UPLC)), by filtration, by electrophoresis (e.g., gel electrophoresis), by thin layer liquid chromatography, ultrafiltration and/or by precipitation.

Incubating may be incubating by any means. Preferably, incubating is incubating in an aqueous solvent in particular an aqueous buffer enabling the enzymatic activity of the polypeptide or derivative thereof of the present invention.

In a preferred embodiment, the step (iii) of incubating the mixture comprises incubating at conditions wherein at least a fraction of acetylated xanthan has a disordered conformation, preferably wherein said fraction constitutes at least 5 % (w/w), more preferably at least 10 % (w/w), even more preferably at least 20 % (w/w), even more preferably at least 50 % (w/w), even more preferably at least 80 % (w/w) of the total content of xanthan.

In this context, the acetylated xanthan used in the context of the present invention may be entirely ordered, partly disordered or entirely disordered as can be measured by circulardicroism (CD) spectroscopy. Preferably, at least a fraction of acetylated xanthan has a disordered conformation. The terms "disordered" and "open" conformation may be understood interchangeably. This is the conformation that automatically is formed in increasing amounts in the xanthan upon employing high temperatures and/or low concentrations of dissolved molecules, in particular low salt concentrations.

The presence of a disordered conformation is of particular benefit when an enzyme cleaving off the inner mannose, in particular an acetyl xylan esterase as described above is used in the method.

Disordering the xanthan conformation may be achieved by any means known in the art. Therefore, exemplarily, the temperature at which the xanthan is incubated with the enzymes may be above a certain threshold and/or the concentration of dissolved molecules may be below a certain threshold.

When performing the method of the present invention, incubating the mixture may be performed at any temperature above a certain threshold still suitable for the enzymes of the present invention to be active.

Accordingly, in a preferred embodiment, the step (iii) of incubating the mixture comprises incubating at a temperature of at least 10°C, preferably of at least 20°C, more preferably of at least 30°C in particular of 40°C or higher.

The temperature may be constant over the entire incubation period or may vary over time. The incubation time required for the desired result will depend on the degree of altering the acetylation pattern of the xanthan to be achieved, the concentration of the polypeptide or derivative of the present invention employed, the specific activity of the polypeptide or derivative of the present invention employed, the concentration of the xanthan, the degree and pattern of acetylation of the substrate xanthan, the pH, the salt concentration, the co-factor concentrations, the presence of potential inhibitors and not the least the temperature.

Additionally or alternatively, in a preferred embodiment, the step (iii) of incubating the mixture comprises, incubating in an environment comprising a total concentration of dissolved molecules other than xanthan and enzymes of not more than 100 mM, more preferably of not more than 75 mM, even more preferably of not more than 50 mM, even more preferably of not more than 25 mM, in particular incubating in an environment essentially free of dissolved molecules other than xanthan and enzymes.

Essentially free of dissolved molecules other than xanthan and enzymes preferably means that the total concentration of said molecules is not higher than 10 mM, more preferably not higher than 5 mM, even more preferably not higher than 4 mM, even more preferably not higher than 3 mM, even more preferably not higher than 2 mM, even more preferably not higher than 1 mM, even more preferably not higher than 0.5 mM, even more preferably not higher than 0.2 mM, in particular not higher than 0.1 mM.

This concentration-dependent effect of dissolved molecules on the deacetylation yields may be also found for some of the employable enzymes only. Exemplarily for enzyme such as, e.g., AXE3, salt concentrations may influence the reaction whereas they may have a far lower influence on other enzymes such as, e.g., YesY. So, YesY may work nicely on xanthan having a closed conformation as is typically found in a salt-containing environment.

In this context, particularly, the molecules other than xanthan and enzymes may be dissolved salt ions and/or small molecular (i.e., having a MW < 500 Da) organic molecules (e.g., glycerol, dimethyl sulfoxide (DMSO) and/or sugars). It will be apparent that cofactors of the enzymes, even when having a MW not more than 500 Da may nevertheless be optionally present during incubation.

Accordingly, in particular the salt concentration present during incubation may influence the efficacy of the enzyme(s) of the present invention. It may be below 1 mM, between 1 and 10 mM, between 10 and 50 mM or between 50 and 100mM.

The step of incubating may be performed as long as required to obtain the xanthan with altered acetylation pattern of interest.

Typically, the step (iii) of incubating the mixture comprises incubating for at least one hour, preferably for at least five hours, more preferably for at least ten hours, in particular for at least a day.

When incubating, the pH may be in any range suitable for the polypeptide or derivative thereof of the present invention.

In a preferred embodiment, the step (iii) of incubating the mixture comprises incubating at an essentially neutral pH.

An essentially neutral pH is a pH between pH 5 and 9, preferably between pH 6 and 8, more preferably between pH 6 and 7.8, even more preferably between pH 6.5 and 7.5, in particular around pH 7.

The xanthan obtainable from the method of the present invention bears special technical properties, e.g., improved rheology properties of solutions compared to the educt xanthan.

Therefore, in a further aspect, the present invention relates to xanthan obtainable from a method of the present invention as described in detail above.

In the context of the xanthan of the present invention, the definitions given in the context of the method above also apply.

As mentioned above, this xanthan has an altered acetylation pattern. This may be any altered acetylation pattern.

In a preferred embodiment, in the xanthan according to the present invention at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner mannose moieties are deacetylated, in particular wherein essentially all inner mannose moieties are deacetylated, optionally wherein the outer mannose moieties are essentially not deacetylated or also deacetylated.

Therefore, in a more preferred embodiment, in the xanthan according to the present invention at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner mannose moieties are deacetylated, in particular wherein essentially all inner mannose moieties are deacetylated, wherein the outer mannose moieties of said xanthan are essentially not deacetylated.

In an alternative embodiment, in this xanthan, also the outer mannose moieties of said xanthan are essentially deacetylated.

In a further preferred embodiment, in the xanthan according to the present invention at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the outer mannose moieties are deacetylated, in particular wherein essentially all outer mannose moieties are deacetylated, optionally wherein the inner mannose moieties are essentially not deacetylated or also deacetylated.

Therefore, in a more preferred embodiment, in the xanthan according to the present invention at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the outer mannose moieties are deacetylated, in particular wherein essentially all outer mannose moieties are deacetylated, wherein the inner mannose moieties of said xanthan are essentially not deacetylated.

In an alternative embodiment, in this xanthan, also the inner mannose moieties of said xanthan are essentially deacetylated.

In a particularly preferred embodiment, in the xanthan according to the present invention at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner and outer mannose moieties are deacetylated, in particular wherein essentially all inner and outer mannose moieties are deacetylated.

In an alternatively preferred embodiment, in the xanthan according to the present invention not more than 5 %, not more than 10 %, not more than 20%, not more than 30%, not more than 40% or not more than 50 of the inner and outer mannose moieties are deacetylated, in particular wherein between about 5 and about 90 % or between about 5 and about 50 % or between about 10 and about 70 % of the acetyl moieties are removed from xanthan are deacetylated.

As mentioned above, the xanthan according to the present invention bears special technical properties, e.g., improved rheology properties of solutions compared to the educt xanthan and to xanthans known in the art.

Depending on the enzymes employed in the method, the incubation conditions (e.g., incubation time, incubation temperature, total concentration of dissolved molecules in the used buffer, pH of the used buffer) and the ratio of enzymes to another, the person skilled in the art being aware of the present invention is enabled to produce xanthan with a well-defined acetylation pattern. Herein, the person skilled in the art will be able to chose a special enzyme composition and adjust the incubation conditions accordingly to obtain the desired xanthan. Many of these xanthans with special characteristics have novel and improved properties not found in the art so far.

Therefore, in a further aspect, the present invention refers to xanthan, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner mannose moieties are free of acetyl residues, in particular wherein essentially all inner mannose moieties are free of acetyl residues, optionally wherein at least 10% or at least 50% of the outer mannose moieties bear at least one acetyl residue.

In a preferred embodiment of this aspect, at least 10% or at least 50% of the outer mannose moieties bear at least one acetyl residue.

A still further aspect of the present invention relates to xanthan, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the outer mannose moieties are free of acetyl residues, in particular wherein essentially all outer mannose moieties are free of acetyl residues, optionally wherein at least 10% or at least 50% of the inner mannose moieties bear at least one acetyl residue.

In a preferred embodiment of this aspect, at least 10% or at least 50% of the inner mannose moieties bear at least one acetyl residue.

In a yet further aspect, the present invention relates to xanthan, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner and outer mannose moieties are free of acetyl residues, in particular wherein essentially all mannose moieties are free of acetyl residues.

Accordingly, in a preferred embodiment, the xanthan is essentially free of acetyl residues at specific mannose moieties and/or at all mannose moieties.

This may be understood in the broadest sense. Accordingly, the xanthan may be deacetylated at specific mannose residues only or may further be also deacetylated at other positions. It may even be essentially entirely deacetylated. Preferably, it is essentially deacetylated at specific mannose residues and still bears acetyl residues at other positions. The xanthan may be deacetylated at the inner mannose residues and still bear acetyl residues at other positions including the outer mannose. Alternatively, the xanthan may be deacetylated at the outer mannose residues and still bear acetyl residues at other positions including the inner mannose. Alternatively, the xanthan may be deacetylated at the inner and outer mannose residues and still bear acetyl residues at other positions.

Due to its improved technical properties, the xanthan obtainable from the method of the present invention, said xanthan also has an improved applicability in a plenty of technical applications.

Accordingly, a yet further aspect of the present invention relates to the use of the xanthan according to the present invention for modifying rheology properties, viscosity properties, thickening, gelling, preventing the formation of ice crystals, stabilizing suspensions and/or emulsions, preserving, moistening and/or hydrating skin.

In the context of the use of the xanthan of the present invention, the definitions given in the context of the use, method and xanthan obtainable from said method above also apply.

In a preferred embodiment, the xanthan according to the present invention is for use in

(i) an ingestible, in particular gluten-free bakery products, frozen food, dairy products, liquid food, beverages and/or water-based recipes;
(ii) a personal care product, in particular a tooth-paste and/or cosmetics;
(iii) a cleaning agent;
(iv) an agent for drilling, in particular for drilling oil;
(v) an agrochemical formulation;
(vi) an additive to solid material, in particular ceramics and/or a construction material; and/or
(vii) a paint.

Herein, frozen food may be exemplarily frozen vegetable, frozen fruits, ice cream, frozen fish, frozen meat and/or frozen pre-packed food.

Ingestibles may be any edible or drinkable formulations known in the. A liquid food may comprise soups, (salad) dressings, sauces and any other solutions, emulsions or suspensions used in ingestibles.

A cleaning agent may be any liquid cleaning agent known in the art such as, e.g., a toilet cleaner, a dishwashing agent, a dish liquid, a laundry agent, a fabric conditioner, a softener, a light-duty agent, a dish washer additive, a cleansing agent and/or a polishing agent.

According to the general inventive concept of the present invention, in a further aspect, the present invention relates to the use of an enzyme site-specifically cleaving off the acetyl residues off mannose moieties of acetylated xanthan.

In this context, in a preferred embodiment, the method of the present invention a laid out above is used.

Accordingly, in the context of the use, the definitions given in the contexts of the method and xanthan above also apply.

The following figures and example are intended to illustrate the invention but not to limit the scope of protection conferred by the claims.

**Figures**

**Figure 1** exemplifies a general structure of xanthan. Figure 1A depicts the xanthan repeating unit as described by Kool et al. (Kool et al., 2013b). This structure shows, from top to bottom, the two glucose moieties forming the backbone of the xanthan, the inner mannose moiety, the glucuronic acid moiety and the outer mannose moiety. Figure 1B depicts the six xanthan repeating units, as identified by HILIC-ELSD-ESI-IT-MSn.

**Figure 2** depicts the Expression Production of the YesY enzyme. Herein, Figure 2A depicts the Expression plasmid pET22b::YesY-StrepIIc. The sequence coding for the C-terminally StrepII-tagged YesY is highlighted. Relevant structural elements are indicated: F1 ori, origin of replication; *bla,* β-lactamase gene; lacI, lacI repressor gene. Figure 2B depicts gels of an SDS-PAGE (left) and a Western blot analysis (right) of FPLC-purified YesY and muteins. 30 μg of crude extract (CE) and 8 μg of purified enzyme (pure) were loaded onto an SDS gel and electrophoretically separated. The gel was stained using Coomassie brilliant blue. For the corresponding Western blot a Strep-Tactin HRP conjugate was used to detect the StrepII-tagged enzyme.

**Figure 3** shows the correlation between the xanthan conformation and the enzymatic degradation by cellulases. Diamonds: normal xanthan; triangles: acetyl-free xanthan; dots: pyruvate-free xanthan; squares: acetyl- and pyruvate-free xanthan.

**Figure 4** depicts the acetic acid release from xanthan after treatment with different carbohydrate acetyl esterases after a 48 h-incubation at 40°C at different solvent conditions.

**Figure 5** depicts far UV circular dichroism (CD) spectra of xanthan in 0, 1, 2, 5, and 10 mM NaCl solutions at 40°C. $\alpha$ = the fraction of disordered conformation.

**Figure 6** shows the influence of the xanthan conformation on the acetic acid release by AXE3. Here, acetic release from xanthan obtained after incubation with AXE3 for 48 h at different fraction of disordered conformation ($\alpha$ = the fraction of disordered conformation) is shown.

**Figure 7** demonstrates that the temperature dependency of the deacetylating activity of AXE3 towards xanthan (diamonds) differing from the one towards xylan (squares). Notably, the temperature optima differ from another.

**Figure 8** depicts the AXE3 modification of xanthan followed in time. Herein, Figure 8A depicts the HILIC-ELSD elution profile of unmodified xanthan and xanthan modified with AXE3 for 1 day, 2 days or 3 days. Figure 8B shows the relative abundance of the six xanthan repeating units at different levels of AXE three modification square: acetylated and pyruvulated RU; triangles: single inner mannose acetylated RU; diamonds: double acetylated RU; dots: pyruvylated RU; crosses: unsubstituted RU; stars: single outer mannose acetylated RU.

**Figure 9** depicts the enzyme : substrate ratio of YesY for deacetylation of xanthan.

**Figure 10** depicts the pH and temperature optima of the YesY enzyme. Herein, Figure 10A depicts the pH optimum and Figure 10B depicts the temperature optimum of the YesY enzyme.

**Figure 11** shows the comparison of the xanthan structure before and after modification with YesY (line below = unmodified xanthan; upper line = YesY modified xanthan)

**Figure 12** depicts the mass spectrometry (MS-MS) $MS^2$-fragmentation pattern of the single acetylated repeating unit (eluting at 12.5 min in Figure 8A) of unmodified xanthan and 3 days AXE3 modified xanthan. Herein, ¤ refers to fragments specific for the acetylation on the outer mannose and * refers to fragments specific for the acetylation of the inner mannose.

**Examples**

## I. General Materials and Methods used Herein

1. Chemicals and substrates

All chemicals used were, if not mentioned otherwise, of analytical grade. The xanthan used was kindly provided by DuPont (Melle, France) and characterised as described previously (Kool et al 2013a). Ninety per cent the inner mannose units are acetylated; 66% of the outer mannose units are pyruvylated and 21% of the outer mannose units are acetylated.

2. Circular Dichroism

The far-UV CD spectra of 2 mg•mL-1 xanthan in 0, 1, 2, 5 and 10 mM NaCl solutions were measured at 20, 40 and 70°C using a Jasco-J-715 spectro-polarimeter, model J-715 (JASCO, Tokyo, Japan). A quartz cuvette with an optical path of 1 mm was used. The temperature was regulated using a Jasco PTC-348 WI controller (JASCO, Tokyo, Japan). In the 190-300 nm wavelength region (0.2 nm resolution) 10 scans were accumulated with a scan rate of 100 nm•min-1 and a time constant of 0.125 s. The final spectra are the average of these scans. Prior to the spectral analysis the wavelength scans were corrected for the buffer background signal.

Previous research showed that the decrease in ellipticity (θ) at 219 nm is almost linear with the fraction of xanthan that appears in the disordered conformation (Kool et al., 2013a). The fraction of disordered conformation (α) was, therefore, estimated using Equation 1 with: θs= ellipticity of the sample at 219 nm; θU = ellipticity of a completely disordered structure at 219 nm and θF = ellipticity of a completely ordered structure at 219 nm.

$$\alpha = (\theta s - \theta F) / (\theta U - \theta F) \qquad (1)$$

The ellipticity of θF was determined in 10 mM NaCl solutions at 15°C and the ellipticity of θU was determined in milliQ water 70°C.

## II. Production of the Enzymes

1. Production of YesY

The open reading frame coding for YesY was amplified using the primers YesY-for (5'-GCAATTCCATATGGCAAATCATATTTATCTTGCCG-3') and YesY-rev (5'-CTCCCTTTCTTTGATGATCGATTC-3'), genomic DNA from *Bacillus subtilis* strain 168 as the template, and a proofreading DNA polymerase (Phusion, Finnzymes). *Bacillus subtilis* strain 168 is commonly available from the Leibniz Institute DSMZ-German Collection of Microorganisms and Cell Cultures (DSMZ, Braunschweig). YesY-for introduced an NdeI restriction endonuclease recognition site overlapping with the start codon and YesY-rev introduced a SacI recognition site, removing the stop codon at the same time.

The amplicon was kit-purified, cut with NdeI and SacI, and ligated with the likewise cut and dephosphorylated expression vector pET22b-StrepIIc. Such vector originates from pET-22b(+) (Novagen) and contains an additionally introduced sequence coding for the StrepII affinity tag. A scheme of the generated expression plasmid pET22b::YesY-StrepIIc is shown in Figure 2A. The expression strain *E. coli* Rosetta2 (DE3)[pLysSRARE2] was transformed with the expression plasmid and the thus generated strain was used to produce YesY. For this the strain was grown in 500 mL auto-induction medium (Studier 2005) complemented with ampicillin (f.c. 100 μg/mL) and chloramphenicol (f.c. 34 μg/mL). Incubation was done at 37°C for 4 h at 180 rpm and continued at 26°C for 20 h at 120 rpm. Then the cells were harvested by centrifugation and the cell pellet was resuspended in FPLC buffer (40 mM triethanolamine, 400 mM NaCl, pH 8.0) and frozen/stored over-night at -20°C. After melting, the cells were opened by intracellular lysozyme activity and by ultrasonic treatment. Then the suspension was centrifuged (45 min, 40.000 x g, 4°C) and the supernatant (= crude extract) was subjected to FPLC using a Strep-Tactin column (Qiagen). The StrepII-tagged YesY (26.3 kDa) was purified, re-buffered, and concentrated using Vivaspin columns (10.000 MWCO; Sartorius). From a 500-mL culture up to 5 mg of highly pure YesY were obtained. To verify purity and integrity of the enzyme an SDS-PAGE analysis and a Western blot were performed (Figure 2B). YesY muteins, carrying single amino acid exchanges, were likewise analyzed.

2. Production of AXE3 and AXE2

The AXE3 and AXE2 enzymes were produced as shown in Pouvreau et al., 2011. Accordingly, the ascomycetous fungus *C. lucknowense* C1 (accession number: VKM F-3500D of the All-Russian Collection of Microorganisms of Russian Academy of Sciences) was used for the production of hemicellulases. The full genome of C1 has been sequenced and revealed many genes encoding for cellulases, hemicellulases and accessory enzymes. Annotated genes, encoding for the putative acetyl xylan esterases Axe2 and Axe3, have been cloned into a specially designed C1-expression host that allows production of relatively pure enzyme. The amino acid sequences have been deposited at GenBank with the accession numbers HQ324256 (Axe2) and HQ324257 (Axe3). The strains, containing extra copies of the axe2 or axe3 gene were grown aerobically in 2 L fermentors in mineral medium, containing glucose as carbon source, ammonium sulfate as nitrogen source and trace elements for the essential salts. Growth is performed under glucose limitation at pH 6.0 and 32 °C.

**III. Overview of all carbohydrate acetyl esterases tested**

**Table 1. Overview of all carbohydrate acetyl esterases tested**

| enzyme code | Accession number | Origin | Activity known/supposed by literature | Cazy family | Corresponding literature |
|---|---|---|---|---|---|
| AXE2 | HQ324256 | *Myceliophthora thermophila C1* | Xylan | CE-5 | Pouvreau et al., 2011; Hinz et al, 2009 |
| AXE3 | HQ324257 | *Myceliophthora thermophila C1* | Xylan | CE-1 | Pouvreau et al., 2011; Hinz et al, 2009 |
| AXE6 | AAU39337.1 | *Bacillus licheniformis* DSM13 | Cephalosporin C deacetylase | CE-7 | - |
| AXE7 | CAB12112.1 | *Bacillus subtilis* str. 168 | Acetyl xylooligo-saccharide esterase | CE-7 | - |
| AXE8 | YP_006663358.1 | *Arthrobacter nitroguajacolicus* Rü61a | Cephalosporin C deacetylase | unknown | - |
| CDA-II | AAU39149.1 | *Bacillus licheniformis* DSM13 | Polysaccharide deacetylase | CE-4 | - |
| CDA-III | AAU39762.1 | *Bacillus licheniformis* DSM13 | Polysaccharide deacetylase | CE-4 | - |
| PAE2 | CAG75311.1 | *Pectobacterium carotovorum* | Pectin | unknown | - |
| PAE4 | AAU42913.1 | *Bacillus licheniformis* DSM13 | Pectin: Rhamnogalacturonan | CE-12 | - |
| PAE5 | AAU40277.1 | *Bacillus licheniformis* DSM13 | Pectin: Rhamnogalacturonan | CE-12 | - |
| PPDA | NP_626539.1 | *Streptomyces coelicolor* A3(2) | Acetyl xylan esterase | CE-4 | |
| YesY | NP_388588.1 | *Bacillus subtilis* str. 168 | Pectin: Rhamnogalacturonan | CE-12 | Ochiai et al., 2007 |
| YesT | NP_388583.1 | *Bacillus subtilis* str. 168 | Xylan, Pectin: Rhamnogalacturonan | CE-12 | Ochiai et al., 2007 |
| RG-04 | unknown | *Aspergillus aculeatus* | Pectin: Rhamnogalacturonan | CE-12 | Searle-van Leeuwen at al., 1992 |

(continued)

| enzyme code | Accession number | Origin | Activity known/supposed by literature | Cazy family | Corresponding literature |
|---|---|---|---|---|---|
| PE-04 | unknown | *Aspergillus niger* | Pectin: Homogalacturonan | unknown | Searle-van Leeuwen et al., 1996 |
| R-06 | unknown | *Aspergillus niger* | Xylan: Birch wood | CE-1 | Kormelink et al., 1993 |

Conclusions

In this research nine known carbohydrate acetyl esterases were screened for their activity towards xanthan. Acetyl Xylan Esterase 3 (AXE3) produced by Myceliophthora thermophila C1 from CE family 1, is active towards xanthan, but only towards the disordered conformation. Characterisation of the modified xanthan showed that AXE3 is specific for the removal of the acetyl groups positioned on the inner mannose unit. After a 3-day incubation ~77% of all acetyl groups on the inner mannose are removed. Although the specific activity of AXE3 towards xanthan is much lower compared to the activity towards xylan, we thereby report the first acetyl esterase that is active towards xanthan.

As it is shown that the disordered conformation is necessary for the removal of acetyl groups from the inner mannose, future research towards xanthan acetyl esterases should focus on thermophilic AEs or on AEs which are stable and active in a salt free environment. Furthermore it is recommended to start screening of known AEs which have an a-specific binding site or which are member of CE family 2, since these AEs are specific for the deacetylation of carbohydrates at the O-6 position.

**IV. Use of Exemplified Enzymes**

1. Analysis of a set of Enzymes

A set of the enzymes enlisted above were used in a number of enzymatic assays.

1.1. Enzyme assays

An overview of all carbohydrate acetyl esterases used, their origin and their known specificity is given in Table 2.

**Table 2. Characteristics of the carbohydrate acetyl esterases used in this study, including abbreviation, CAZY family and references**

| Activity | Abbr. | Origin | Known activity | CAZy Family | Reference |
|---|---|---|---|---|---|
| Acetyl xylan esterase | AXE2 | *Myceliophthora thermophila C1* | Xylan O2 and O3 position | CE-5 | Pouvreau (2011); Hinz (2009) |
| | AXE3 | *Myceliophthora* | Xylan | CE-1 | Pouvreau |
| | | *thermophila C1* | | | (2011); Hinz (2009) |
| | AnAXE | *Aspergillus niger* | Xylan | | Kormelink (1993) |
| | | | | | |
| Chitin deacetyl ase | CDA-II | | Chitin | CE-4 | - |
| | CDA-III | | Chitin | CE-4 | |
| | | | | | |
| Pectin acetyl esterase | PAE2 | *Pectobacterium carotovorum* | Pectin | | - |

(continued)

| Activity | Abbr. | Origin | Known activity | CAZy Family | Reference |
|---|---|---|---|---|---|
| | PAE4 | *Bacillus licheniformis* | Pectin Homogalacturonan | | - |
| | RG-04 | *Aspergillus aculeatus* | Pectin: Rhamnogalacturonan | CE-12 | Searle-van Leeuwen (1992) |
| | PE-04 | *Aspergillus niger* | Pectin: Homogalacturonan | 12/13 | Searle-van Leeuwen (1996) |

AXE2 and AXE3 (Hinz et al., 2009; Pouvreau, Jonathan, Kabel, Hinz, Gruppen and Schols, 2011) were kindly provided by Dyadic (Wageningen, The Netherlands). CDA-II, CDA-III, PAE2, PAE4, RG-04, PE-04 and AnAXE were expressed and purified as described elsewhere (Kormelink, Lefebvre, Strozyk and Voragen, 1993; Searle-van Leeuwen, Broek, Schols, Beldman and Voragen, 1992; Searle-van Leeuwen et al., 1996). All enzymes were desalted shortly before testing their activity towards xanthan using Micro Bio-Spin chromatography columns following the companies description (Bio-Rad Laboratories, Hercules, CA, USA).

The carbohydrate acetyl esterases were screened for their activity towards xanthan by incubating one mL of a 2 mg•mL-1 xanthan solution with 8-35 μg enzyme at 40°C for 48 h. The enzyme activity was tested in: (1) Millipore water, (2) 10mM NaCl solutions and (3) 50 mM citric acid buffer (pH 6). The acetic acid release was determined using a Megazyme acetic acid kit (Megazyme, Wicklow, Ireland) and expressed as percentage of the total acetic acid concentration. The protocol was downscaled to microtiter scale to enable high throughput analysis. All incubations were performed in duplicates.

Acetyl esterase AXE3, that showed activity towards xanthan, was further characterised. The precise influence of the xanthan conformation on the enzyme activity was determined by incubating 2 mg•mL-1 xanthan in Millipore water, 1 mM, 2 mM, 5mM and 10mM NaCl solution with 25 μg of protein at 40°C for 48 h. The temperature optimum was determined in a salt free environment within the temperature range 35-60°C after a 24 h-incubation. All incubations were performed in fourfold.

The mode of action of the AXE3 and YesY towards xanthan was determined by structural analysis of unmodified and enzymatically modified xanthan. The modified xanthan solutions were dialysed against water and lyophilized. The obtained xanthan was redissolved in Millipore water (2 mg•mL-1) and incubated with the cellulases from Myceliophthora thermophila C1 (Dyadic Netherlands, Wageningen, The Netherlands) at 60°C for 48h (Kool at al., 2013a). The obtained xanthan digests were analysed for their repeating units using HILIC-ELSD-ESI-IT-MSn as described previously (Kool et al., 2013b).

<u>1.2. Results and Discussion</u>

<u>Screening for xanthan acetyl esterase activity</u>

The three-dimensional conformation of xanthan depends on the ionic strength and temperature of a xanthan solution (Bezemer, Ubbink, Kooker de, Kuil and Leyte, 1993; Matsuda, Biyajima and Sato, 2009). As it is known that the enzymatic hydrolysis of the xanthan backbone by cellulases strongly depends on this conformation (Kool at al., 2013a; Rinaudo and Milas, 1980), the enzyme activity of all AEs was tested at different solvent conditions. An overview of the acetic acid release from xanthan after 48 h of incubation with the different AEs is shown in Figure 4.

The results show that most of the AEs are not active towards xanthan. AXE3 is the only AE that released significant amounts of acetic acid from xanthan, however this enzyme showed no activity in 10mM NaCl solutions and/or in 50mM Citric acid buffer. Because the ordered helical conformation is favoured with increasing ionic strength (Morris, Rees and Young, 1977), it is likely to assume that AXE3 is only active towards the disordered xanthan conformation, possibly because the acetyl groups are only accessible when xanthan appears in the disordered conformation. If acetyl groups are indeed only accessible in the disordered conformation, only those acetyl esterases which are heat stable and/or active in a salt free environment are likely to be active towards xanthan. The activity of all AEs towards their model substrate (acetylated sugar beet pectin, acetylated xylan oligosaccharides or chitin oligosaccharides) was therefore tested in a salt free solution at 40°C. None of the enzymes, except AXE3, was active at such conditions (results not shown). Assuming that the disordered conformation is indeed required for the enzymatic deacetylation of xanthan, this explains why, except for AXE3, none of the other tested AEs is active towards xanthan.

Previous research towards AXE3 and AXE2 showed that both enzymes, although from different CE families, showed similar activity towards acetylated xylan oligosaccharides (Hinz et al., 2009; Pouvreau, Jonathan, Kabel, Hinz, Gruppen and Schols, 2011). AXE3 had a lower initial activity, however, the total amount of acetic acid released from acetylated xylan oligosaccharides at the end point was similar for both enzymes. It is therefore a bit unexpected that these enzymes show a different activity towards xanthan. It seems that AXE2 is hindered by the xanthan structure, whereas AXE3 is not. As described by Pouvreau (2011) AXE2 was, in contrast to AXE3, not active towards the synthetic substrate p-nitrophenyl acetate. It was hypothesized that the two enzymes have a different binding site, and that AXE3 has a less specific binding site since it only requires an acetyl group for binding (Pouvreau, Jonathan, Kabel, Hinz, Gruppen and Schols, 2011). Further activity tests towards different acetylated xylan substrates indeed showed that AXE2 and AXE3 are differently influences by the size and structure of a substrate (Pouvreau, Jonathan, Kabel, Hinz, Gruppen and Schols, 2011). More general studies towards acetyl xylan esterases from different CE families showed that enzymes from CE family 4 and 5 indeed have a higher specificity than acetyl esterases from CE family 1 (Biely, 2012).

The acetyl groups in xanthan on both the inner as well as the outer mannose unit are linked at the O-6 position. Substrate specificity tests performed on CE family 2 indicated that this specific CE family preferably deacetylates polysaccharides and/or oligosaccharides at the O-6 position (Biely, 2012; Topakas, Kyriakopoulos, Biely, Hirsch, Vafiadi and Christakopoulos, 2010). In this study no AEs from CE family 2 were tested, but further screening for xanthan acetyl esterases might focus on known AEs from this specific CE family.

Influence of the xanthan conformation on the acetic acid release by AXE3

To be able to analyse the influence of the xanthan conformation on the AXE3 activity, the conformation of xanthan at 40°C in solutions with different NaCl concentrations was estimated using circular dichroism (CD) and Equation 1. The far UV CD spectra are shown in Figure 5.

Figure 6 shows the acetic acid release from xanthan after a 48 h incubation with AXE3 at different $\alpha$. It is shown that with an increasing fraction of disordered conformation, more acetic acid is released. It is therefore concluded that AXE3 indeed is only active towards disordered xanthan molecules. Several studies on the xanthan conformation argued that the acetyl groups positioned on the inner mannose unit show interaction with the xanthan backbone and are, therefore, positioned at the inside of the xanthan helix (Morris, Rees and Young, 1977; Pelletier, Viebke, Meadows and Williams, 2001; Tako and Nakamura, 1984). These acetyl groups would therefore not by accessible for acetyl esterases when xanthan is in an ordered conformation, which explains that only the disordered xanthan structures are deacetylated by AXE3. In a recent study we showed that ~20% of all acetyl groups are positioned on the outer mannose (Kool et al., 2013b). Whether these acetyl groups also fold to the inside of the helical conformation is unknown. However it is known that xanthan lyases are able to remove the outer mannose in the presence of salts (Hashimoto, Miki, Tsuchiya, Nankai and Murata, 1998; Ruijssenaars, de Bont and Hartmans, 1999). It is, therefore, likely to assume that the outer mannose is accessible for enzymes when xanthan appears in an ordered conformation. The acetyl groups on the outer mannose unit should, therefore, be accessible for enzymes independent of the xanthan conformation. Since AXE3 is only active towards disordered xanthan fragments, AXE3 could be specific for the removal the acetyl groups from the inner mannose unit. This indicates that, when screening for xanthan acetyl esterases, it is off great importance to test the activity of the enzyme in conditions supporting the disordered as well as the ordered xanthan conformation in order to not overlook any deacetylating activity.

Characterisation of the AXE3 activity towards xanthan

Temperature optimum:

The temperature optimum of AXE3 towards xanthan was determined in a temperature range from 35-60°C in a salt free environment and compared to the temperature optimum observed towards acetylated xylan oligosaccharides at pH 7 (Pouvreau, Jonathan, Kabel, Hinz, Gruppen and Schols, 2011). The results are shown in Figure 7. AXE3 has a clear temperature optimum towards xanthan between 50-55°C, whereas a broader and lower temperature optimum (35-45°C) was observed towards xylan oligosaccharides at pH 7. Because the xanthan conformation changes to a more disordered structure with increasing temperature, the temperature optimum observed for xanthan is a compromise between the optimal temperature for the enzyme and the total amount of accessible acetyl groups. The observed difference in temperature optima towards the two substrates can, therefore, be explained by the influence of the conformational behaviour of xanthan on the enzyme activity.

Specific activity:

To compare the activity of AXE3 towards xanthan with the activity towards xylan, the specific activity of AXE3 towards xanthan was determined after a one day incubation at 55°C in a salt free environment. The specific activity towards acetylated xylan oligosaccharides (pH 7; 40°C), as determined by Pouvreau (2011) is 8.3 U·mg protein-1. Towards xanthan the specific activity is 13 mU·mg protein-1. So although the enzyme can remove acetyl groups from xanthan, the activity is really low compared to its main activity. We, therefore, conclude that the observed activity towards xanthan is only a side activity of AXE3 and that the original annotation as acetyl xylan esterase is still correct.

Characterisation of the modified xanthan:

To analyse the xanthan structure the enzymatically modified xanthan, xanthan modified with AXE3 for 1 day, 2 days or 3 days was degraded to its repeating units using cellulases. The relative abundance of the repeating units present in the modified xanthans was determined using HILIC-ELSD-MS (Kool et al., 2013b). The obtained HILIC elution profiles were compared to the profile of unmodified xanthan (Figure 8). After a one day modification with AXE3 the double acetylated RUs and the acetylated and pyruvylated RUs are decreased slightly in their abundance. The amount of single acetylated RUs, unsubstituted RUs and pyruvylated RUs slightly increased. Extending the AXE3 modification resulted in a further increase of the unsubstituted RUs and pyruvylated RUs, while all the other RUs decrease. After a 3 day modification with AXE3 almost all double acetylated RUs and acetylated and pyruvylated RUs are modified. However single acetylated RUs remain present even, after a 3 day modification with AXE3. Because both the inner as well as the outer mannose unit can be acetylated and because AXE 3 if only active towards the disordered xanthan conformation, AXE3 is hypothesized to be specific for the deacetylation of the inner mannose. The double acetylated repeating unit would then be converted to single acetylated RUs that are acetylated on the outer mannose. Simultaneously, single acetylated RUs that are acetylated on the inner mannose are converted to unsubstituted RUs. Because the two single acetylated RUs elute simultaneously (Kool et al., 2013b), this could explain that no clear decrease in the total amount of single acetylated RUs is observed in the HILIC-ELSD profile. To validate this hypothesis, the MS-fragmentation pattern of the single acetylated RUs before AXE3 modification was compared to the fragmentation pattern of these RUs after 3 days of modification. The results are shown in Figure 12. As described elsewhere (Kool et al., 2013b), some of the m/z-fragments are specific for inner mannose acetylation, while other m/z-fragments are specific for outer mannose acetylation. Figure 12 shows that the ratio in which these specific fragments are present in the fragmentation pattern is different for the unmodified and modified xanthan. Before modification 12% of all single acetylated RUs are acetylated on the outer mannose and after modification this increased to 47%. This indicates that the RUs which are acetylated on the outer mannose accumulate during AXE3 deacetylation. It is, therefore, concluded that AXE3 is indeed specific for the removal of acetyl groups on the inner mannose unit and that the acetyl groups on the outer mannose are not hydrolysed. Structural characterisation of the unmodified xanthan showed that 78% of all acetyl groups are substituted to the inner mannose. After a 3-day incubation 60% of all acetyl groups is removed by AXE3. Since only the acetyl groups from the inner mannose can be hydrolysed by AXE3, it is concluded that after a 3 day modification 77% of all possible acetyl groups are removed.

1.3. Conclusions

In this research nine known carbohydrate acetyl esterases were screened for their activity towards xanthan. Acetyl Xylan Esterase 3 (AXE3) produced by *Myceliophthora thermophila* C1 from CE family 1, is active towards xanthan, but only towards the disordered conformation. Characterisation of the modified xanthan showed that AXE3 is specific for the removal of the acetyl groups positioned on the inner mannose unit. After a 3 day incubation ~77% of all acetyl groups on the inner mannose are removed. Although the specific activity of AXE3 towards xanthan is much lower compared to the activity towards xylan, we thereby report the first acetyl esterase that is active towards xanthan.

As it is shown that the disordered conformation is necessary for the removal of acetyl groups from the inner mannose, future research towards xanthan acetyl esterases should focus on thermophilic AEs or on AEs which are stable and active in a salt free environment. Furthermore it is recommended to start screening of known AEs which have an a-specific binding site or which are member of CE family 2, since these AEs are specific for the deacetylation of carbohydrates at the O-6 position.

2. First use of Enzyme AXE3

2.1. Materials

Xanthan 80 (the standard xanthan as commercially available from Danisco A/S, Copenhagen, Denmark) was used for all enzyme screening assays. Molecular composition below

Xanthan:

| | |
|---|---|
| xanthan type: | Xanthan 80 |
| Glc:Man:GlcA Molar ratio: | 1 : 0.88 : 0.41 |
| acetyl content [w/w%]: | 5.6 |
| Pyruvate content [w/w%]: | 4.4 |

Enzyme AXE3:

AXE3 was produced as described elsewhere (Hinz (2009) Journal of cereal science 50 (3) 318 -323; Pouvreau (2010) enzyme and microbial technology 49 (3) 312-320)

2.2. Enzyme assays

AXE3 was screened for its activity towards xanthan by incubating one mL of a 2 mg·mL$^{-1}$ xanthan solution with 25 μg enzyme at 40°C for 48 h. The enzyme activity was tested in: Millipore water; in 10mM NaCl solutions and in 50 mM citric acid buffer (pH 6). The acetic acid release was determined using a Megazyme acetic acid kit (Megazyme, Wicklow, Ireland) and expressed as percentage of the total acetic acid concentration. All incubations were performed in duplicates.

The influence of the xanthan conformation on the activity of AXE3 was determined by incubating 2 mg·mL$^{-1}$ xanthan in Millipore water, 1 mM, 2 mM, 5mM and 10mM NaCl solution with 25 μg of protein at 40°C for 48 h. At these conditions, the conformation of xanthan was determined to be: 58, 26, 16, 5 and 0% in the disordered conformation respectively.

The temperature optimum was determined in a salt free environment within the temperature range 35-60°C after a 24 h-incubation.

The mode of action of AXE3 was determined by structural analysis of unmodified and enzymatically modified xanthan. The modified xanthan was dialysed against water and lyophilized. The obtained xanthan was resolved in Millipore water (2 mg·mL$^{-1}$) and incubated with the cellulases from *Myceliophthora thermophila C1* (Dyadic Netherlands, Wageningen, The Netherlands) at 60°C for 48h (Kool et al., 2013a). The obtained xanthan digests were analysed for their repeating units using HILIC-ELSD-ESI-IT-MS$^n$ as described previously.

2.3. Results

As shown in Figure 8, an increasing AXE3 activity is observed with an increase in the fraction of disordered xanthan molecules. It is therefore concluded that acetyl groups are only accessible to AXE3 when xanthan appears in a disordered conformation.

pH optimum

As the disordered conformation is required for AXE3 to be active, it was not possible to set the pH of xanthan solution, thus the pH optimum of AXE3 towards xanthan has not been determined.

Temperature optimum

As shown in Figure 12, the pH optimum of AXE3 towards xanthan differs from the pH optimum towards xylan. As elevated temperatures stimulate the disordered xanthan conformation, the difference in temperature optimum towards the different substrates can be explained by the requirement of a disordered xanthan conformation, whereas the activity towards xylan is not hindered by such conformation dependency.

Specific activity of the enzyme

Comparison of the xanthan structure before and after modification with AXE3 shows that the enzyme specifically removed the acetyl groups on the inner mannose unit.

2.4. Summary of findings for AXE3

AXE3 produced by *Myceliophthora thermophila C1* and characterised in detail to be an example for CE-1 xylan acetyl esterases, could remove 31% of all acetyl groups present in xanthan after an extended incubation at 40°C. Activity towards xanthan was only observed when xanthan molecules were in the disordered conformation, since AXE3 was only active at salt concentrations ≤ 5 mM. Optimal performance of the enzyme towards xanthan was observed 55°C in the complete absence of salt.

Enzymatically modified xanthan was hydrolysed using cellulases and analysed for its repeating units using UPLC-HILIC-ELSD/ESI-MS. It was shown that AXE3 is specific for the removal of the acetyl groups positioned on the inner mannose and that acetyl groups positioned on the outer mannose are not removed at all. After a three day-incubation 80% of all the acetyl esters on the inner mannose were hydrolysed.

3. Use of Enzyme YesY

3.1. Materials

Xanthan 80 (the standard xanthan as commercially available from Danisco) was used for all enzyme screening assays. Molecular composition below

Xanthan:

| | |
|---|---|
| xanthan type: | Xanthan 80 |
| Glc:Man:GlcA Molar ratio: | 1 : 0.88 : 0.41 |
| acetyl content [w/w%]: | 5.6 |
| Pyruvate content [w/w%]: | 4.4 |

Enzyme YesY:

YesY was produced as laid out above.

3.2. Enzyme assays:

YesY was screened for its activity towards xanthan by incubating one mL of a 2 mg·mL$^{-1}$ xanthan solution with different amounts (5.4 - 81 μg) of enzyme at 40°C for 24 h. The enzyme activity was tested in: Millipore water and in 50 mM citric acid buffer (pH 6). The acetic acid release was determined using a Megazyme acetic acid kit (Megazyme, Wicklow, Ireland) and expressed as percentage of the total acetic acid concentration. All incubations were performed in duplicates.

The influence of the xanthan conformation on the activity of YesY was not determined, since the enzyme is active towards the ordered xanthan conformation.

The temperature and pH optimum are not determined using xanthan as substrate. However both are determined with pectin as substrate

- 5 mg SBP3124 was incubated with 4 μg of YesY for 10 min. within the temperature range 40-80°C
- 5 mg SBP3124 was incubated with 4 μg of YesY for 10 min. within the pH range 3-8

The mode of action of YesY was determined by structural analysis of unmodified and enzymatically modified xanthan (1 day modification at 40°C in 50mm citric acid buffer pH 6). The modified xanthan was dialysed against water and lyophilized. The obtained xanthan was redissolved in Millipore water (2 mg·mL$^{-1}$) and incubated with the cellulases from *Myceliophthora thermophila C1* (Dyadic Netherlands, Wageningen, The Netherlands) at 60°C for 48h. The obtained xanthan digests were analysed for their repeating units using HILIC-ELSD-ESI-IT-MS$^n$ as described previously.

SBP 3124 = sugar beet pectin, 31% acetylated, 24% methylated

3.3. Results

Influence of the xanthan conformation on the activity of YesY

The AE activity towards xanthan was tested in milliQ environment and in 50 mM Citric acid buffer pH 6 by incubating 1 mg xanthan with 54 μg YesY for 1 day. Enzyme activity was observed in both conditions, however the highest activity was observed in buffer solution. As the enzyme shows activity in 50 mM citric acid buffer, it is concluded that the enzyme is NOT affected by the xanthan conformation.

After a 24-h incubation in citric acid buffer, 23% of all acetic acid groups is released. Incubation in milliQ resulted in the release of 8% of all acetic acid groups after a 1 day incubation.

The influence of the enzyme: substrate ratio was determined and the results were used to determine the specific activity, which showed to be 0.12 U/mg enzyme.

The pH and temperature optima of the YesY enzyme is depicted in Figure 10.

Specific activity of the YesY towards xanthan

Figure 11 shows the comparison of the xanthan structure before and after modification with YesY (black line = unmodified xanthan; red line = YesY modified xanthan)

All RUs which are acetylated on the inner mannose and pyruvylated on the outer mannose are still present in the xanthan digest after YesY modification. Furthermore the double acetylated repeating units are all degraded by YesY, but the amount of single acetylated RUs increases. This indicates that only the acetyl groups on the outer mannose are removed by YesY. This is as expected, since xanthan appears in an ordered conformation at the chosen enzyme incubations. The acetyl groups on the inner mannose units will therefore not be available for the enzyme.

It was shown that 23% of all acetyl groups in xanthan are removed after a 2-day incubation, indicating that YesY can completely deacetylated the terminal mannose units.

**References**

Bezemer, L., Ubbink, J. B., Kooker de, J. A., Kuil, M. E., and Leyte, J. C. (1993). On the conformational transitions of native xanthan. Macromolecules, 26, 6436 - 6446.

Biely, P. (2012). Microbial carbohydrate esterases deacetylating plant polysaccharides. Biotechnology Advances, 30(6), 1575-1588.

Bradshaw, I. J., Nisbet, B. A., Kerr, M. H., and Sutherland, I. W. (1983). Modified xanthan-its preparation and viscosity. Carbohydrate Polymers, 3(1), 23-38.

Flores Candia, J. L., and Deckwer, W. D. (1999). Effect of the nitrogen source on pyruvate content and rheological properties of xanthan. Biotechnology Progress, 15(3), 446-452.

Hashimoto, W., Miki, H., Tsuchiya, N., Nankai, H., and Murata, K. (1998). Xanthan lyase of Bacillus sp. strain GL1 liberates pyruvylated mannose from xanthan side chains. Applied and Environmental Microbiology, 64(10), 3765-3768.

Hassler, R. A., and Doherty, D. H. (1990). Genetic engineering of polysaccharide structure: Production of variants of xanthan gum in Xanthomonas campestris. Biotechnology Progress, 6(3), 182-187.

Hinz, S. W. A., Pouvreau, L., Joosten, R., Bartels, J., Jonathan, M. C., Wery, J., and Schols, H. A. (2009). Hemicellulase production in Chrysosporium lucknowense C1. Journal of Cereal Science, 50(3), 318-323.

Jansson, P. E., Kenne, L., and Lindberg, B. (1975). Structure of extracellular polysaccharide from Xanthomonas campestris. Carbohydrate Research, 45(DEC), 275-282.

Kool, M. M., Schols, H. A., Delahaije, R. J. B. M., Sworn, G., Wierenga, P. A., and Gruppen, H. (2013a). The influence of the primary and secondary xanthan structure on the enzymatic hydrolysis of the xanthan backbone. Carbohydrate Polymers, 97(2), 368-375.

Kool, M.M., Gruppen, H., Sworn, G., Schols, H.A. (2013b) Comparison of xanthans by the relative abundance of its six constituent repeating units. Accepted for publication in Carbohydrate Polymers, minor revisions - resubmitted June 2013

Kormelink, F. J. M., Lefebvre, B., Strozyk, F., and Voragen, A. G. J. (1993). Purification and characterization of an acetyl xylan esterase from Aspergillus niger. Journal of Biotechnology, 27(3), 267-282.

Matsuda, Y., Biyajima, Y., and Sato, T. (2009). Thermal denaturation, renaturation, and aggregation of a double-helical polysaccharide xanthan in aqueous solution. Polymer Journal, 41 (7), 526 - 532.

Morris, E. R., Rees, D. A., and Young, G. (1977). Order disorder transition for a bacterial polysaccharide in solution. A role for polysaccharide conformation in recognition between Xanthomonas pathogen and its plant host. Journal of Molecular Biology, 110(1), 1-16.

Morrison, N. A., Clark, R., Talashek, T., and Yuan, C. R. (2004). New forms of xanthan gum with enhanced properties. In P. A. Williams, and G. O. Phillips. Gums and Stabilisers for Food Industry 12 pp. 124 - 130). Cambridge: RSC. Pelletier, E., Viebke, C., Meadows, J., and Williams, P. A. (2001). A rheological study of the order-disorder conformational transition of xanthan gum. Biopolymers, 59, 339 - 346.

Ochiai A, Takafumi I, Kawamata A, Wataru H, Murata K. Plant cell wall degradation by saprophytic Bacillus subtilis strains: gene clusters responsible for rhamnogalacturonan depolymerization. 2007; Appl. Environ. Microbiol. 73(12): 3803-3813.

Peters, H. U., Suh, I. S., Schumpe, A., and Deckwer, W. D. (1993). The pyruvate content of xanthan polysaccharide produced under oxygen limitation. Biotechnology Letters, 15(6), 565-566.

Pinto, E. P., Furlan, L., and Vendruscolo, C. T. (2011). Chemical deacetylation natural xanthan (Jungbunzlauer®). Polimeros, 21(1), 47-52.

Pouvreau, L., Jonathan, M. C., Kabel, M. A., Hinz, S. W. A., Gruppen, H., and Schols, H. A. (2011). Characterization and mode of action of two acetyl xylan esterases from Chrysosporium lucknowense C1 active towards acetylated xylans. Enzyme and Microbial Technology, 49(3), 312-320.

Rinaudo, M., and Milas, M. (1980). Enzymic-hydrolysis of the bacterial polysaccharide xanthan by cellulase. International Journal of Biological Macromolecules, 2(1), 45-48.

Ruijssenaars, H. J., de Bont, J. A. M., and Hartmans, S. (1999). A pyruvated mannose-specific xanthan lyase involved in xanthan degradation by Paenibacillus alginolyticus XL-1. Applied and Environmental Microbiology, 65(6), 2446-2452.

Sandford, P. A., Pittsley, J. E., Knutson, C. A., Cadmus, M. C., Watson, P. R., and Jeanes, A. (1977). Variation in Xanthomonas campestris NRRL B-1459; Characterisation of xanthan samples of different pyruvic acid content. Extracellular Microbial Polysaccharides pp. 192-210): American Chemical Society.

Searle-van Leeuwen, M. J. F., Broek, L. A. M., Schols, H. A., Beldman, G., and Voragen, A. G. J. (1992). Rhamnogalacturonan acetylesterase: a novel enzyme from Aspergillus aculeatus, specific for the deacetylation of hairy (ramified) regions of pectins. 38(3), 347-349.

Searle-van Leeuwen, M. J. F., Vincken, J. P., Schipper, D., Voragen, A. G. J., Beldman, G., Visser, J., and Voragen, A. G. J. (1996). Acetyl esterases of Aspergillus niger: purification and mode of action on pectins. Progress in Biotechnology pp. 793-798): Elsevier.

Shatwell, K. P., Sutherland, I. W., Dea, I. C. M., and Ross-Murphy, S. B. (1990). The influence of acetyl and pyruvate substituents on the helix-coil transition behaviour of xanthan. Carbohydrate Research, 206(1), 87-103.

Shatwell, K. P., Sutherland, I. W., Ross-Murphy, S. B., and Dea, I. C. M. (1990). Influence of the acetyl substituent on the interaction of xanthan with plant polysaccharides - I. Xanthan-locust bean gum systems. Carbohydrate Polymers, 14(1), 29-51.

Shatwell, K. P., Sutherland, I. W., Ross-Murphy, S. B., and Dea, I. C. M. (1991). Influence of the acetyl substituent on the interaction of xanthan with plant polysaccharides - II. Xanthan-guar gum systems. Carbohydrate Polymers, 14(2), 115-130.

Smith, C. J. H., Symes, K. C., Lawson, C. J., and Morris, E. R. (1984). The effect of pyruvate on xanthan solution properties. Carbohydrate Polymers, 4, 153-157. Stankowski, J. D., Mueller, B. E., and Zeller, S. G. (1993). Location of a second O-acetyl group in xanthan gum by reductive-cleavage method. Carbohydrate Research, 241(1), 321 - 326.

Tako, M., and Nakamura, S. (1984). Rheology properties of deacetylated xanthan in aqueous-media. Agricultural and Biological Chemistry, 48(12), 2987-2993.

Topakas, E., Kyriakopoulos, S., Biely, P., Hirsch, J., Vafiadi, C., and Christakopoulos, P. (2010). Carbohydrate esterases of family 2 are 6-O-deacetylases. FEBS Letters, 584(3), 543-548.

Visser, H., Joosten, V., Punt, P. J., Gusakov, A. V., Olson, P. T., Joosten, R., Bartels, J., Visser, J., Sinitsyn, A. P., Emalfarb, M. A., Verdoes, J. C. and Wery, J. (2011). Development of a mature fungal technology and production platform for industrial enzymes based on a Myceliophthora thermophila isolate, previously known as Chrysosporium lucknowense C1, Industrial Biotechnology 7 (3): 214-223.

## Claims

1. A method for producing xanthan with an altered acetylation pattern, wherein an enzyme site-specifically cleaving acetyl residues off mannose moieties of acetylated xanthan is contacted with acetylated xanthan.

2. The method of claim 1, wherein the enzyme site-specifically cleaves acetyl residues off the outer mannose moieties of acetylated xanthan,
preferably wherein the enzyme cleaving acetyl residues off the outer mannose is a pectin acetylesterase, preferably a rhamnogalacturonan acetylesterase, more preferably a rhamnogalacturonan acetylesterase of the *yes* cluster of *Bacillus subtilis,* in particular a polypeptide of at least 80% homology to SEQ ID NO: 2 or a functional derivative thereof having at least 10 % of its xanthan-deacetylating activity.

3. The method of claim 1, wherein the enzyme site-specifically cleaves acetyl residues off the inner mannose moieties of acetylated xanthan,
preferably wherein the enzyme cleaving acetyl residues off the inner mannose is an acetyl xylan esterase (AXE), preferably a fungal AXE, more preferably an AXE from *Chrysosporium lucknowense,* in particular a polypeptide of at least 80% homology to SEQ ID NO: 1 or a functional derivative thereof having at least 10 % of its xanthan-deacetylating activity.

4. The method of any one of claims 1 to 3, wherein an enzyme site-specifically cleaving acetyl residues off the inner mannose moieties of acetylated xanthan and an enzyme site-specifically cleaving acetyl residues off the outer mannose moieties of said acetylated xanthan are contacted with acetylated xanthan.

**5.** The method of any one of claims 1 to 4 comprising the steps of:

(i) providing acetylated xanthan,
(ii) contacting said acetylated xanthan with one or more enzyme(s), in particular wherein at least one enzyme is an enzyme according to any of claims 2 or 3,
(iii) incubating the mixture obtained from step (ii) at conditions allowing said enzyme(s) to alter the acetylation pattern of the acetylated xanthan,
(iv) recovering the xanthan with altered acetylation pattern obtained from step (iii).

**6.** The method according to any one of claims 1 to 5, wherein the step (iii) of incubating the mixture comprises:

(a) incubating at conditions wherein at least a fraction of acetylated xanthan has a disordered conformation, preferably wherein said fraction constitutes at least 5 % (w/w), more preferably at least 10 % (w/w), even more preferably at least 20 % (w/w), even more preferably at least 50 % (w/w), even more preferably at least 80 % (w/w) of the total content of xanthan;
(b) incubating at a temperature of at least 10°C, preferably of at least 20°C, more preferably of at least 30°C in particular of 40°C or higher;
(c) incubating in an environment comprising a total concentration of dissolved molecules other than xanthan and enzymes of not more than 100 mM, more preferably of not more than 75 mM, even more preferably of not more than 50 mM, even more preferably of not more than 25 mM, in particular incubating in an environment essentially free of dissolved molecules other than xanthan and enzymes;
(d) incubating for at least one hour, preferably for at least five hours, more preferably for at least ten hours, in particular for at least a day; and/or
(e) incubating at an essentially neutral pH.

**7.** Xanthan obtainable from a method of any one of claims 1 to 6.

**8.** The xanthan according to claim 7, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner mannose moieties are deacetylated, in particular wherein essentially all inner mannose moieties are deacetylated, optionally wherein the outer mannose moieties are essentially not deacetylated or also deacetylated.

**9.** The xanthan according to claim 7, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the outer mannose moieties are deacetylated, in particular wherein essentially all outer mannose moieties are deacetylated, optionally wherein the inner mannose moieties are essentially not deacetylated or also deacetylated.

**10.** The xanthan according to any one of claims 7 to 9, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner and outer mannose moieties are deacetylated, in particular wherein essentially all inner and outer mannose moieties are deacetylated.

**11.** Xanthan, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner mannose moieties are free of acetyl residues, in particular wherein essentially all inner mannose moieties are free of acetyl residues, optionally wherein at least 10% or at least 50% of the outer mannose moieties bear at least one acetyl residue.

**12.** Xanthan, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the outer mannose moieties are free of acetyl residues, in particular wherein essentially all outer mannose moieties are free of acetyl residues, optionally wherein at least 10% or at least 50% of the inner mannose moieties bear at least one acetyl residue.

**13.** Xanthan, wherein at least 10 %, preferably at least 20%, more preferably at least 30%, even more preferably at least 40%, even more preferably at least 50%, even more preferably at least 60%, even more preferably at least 70%, even more preferably at least 80%, even more preferably at least 90% of the inner and outer mannose moieties are free of acetyl residues, in particular wherein essentially all mannose moieties are free of acetyl residues.

**14.** Use of the xanthan according to claim 7 to 13 for modifying rheology properties, viscosity properties, thickening, gelling, preventing the formation of ice crystals, stabilizing suspensions and/or emulsions, preserving, moistening and/or hydrating skin,
in particular for use in

(i) an ingestible, in particular gluten-free bakery products, frozen food, dairy products, liquid food, beverages and/or water-based recipes;
(ii) a personal care product, in particular a tooth-paste and/or cosmetics;
(iii) a cleaning agent;
(iv) an agent for drilling, in particular for drilling oil;
(v) an agrochemical formulation;
(vi) an additive to solid material, in particular ceramics and/or a construction material; and/or
(vii) a paint.

**15.** Use of an enzyme site-specifically cleaving off the acetyl residues off mannose moieties of acetylated xanthan, in particular wherein the method of any of claims 1 to 6 is used.

Fig. 1A

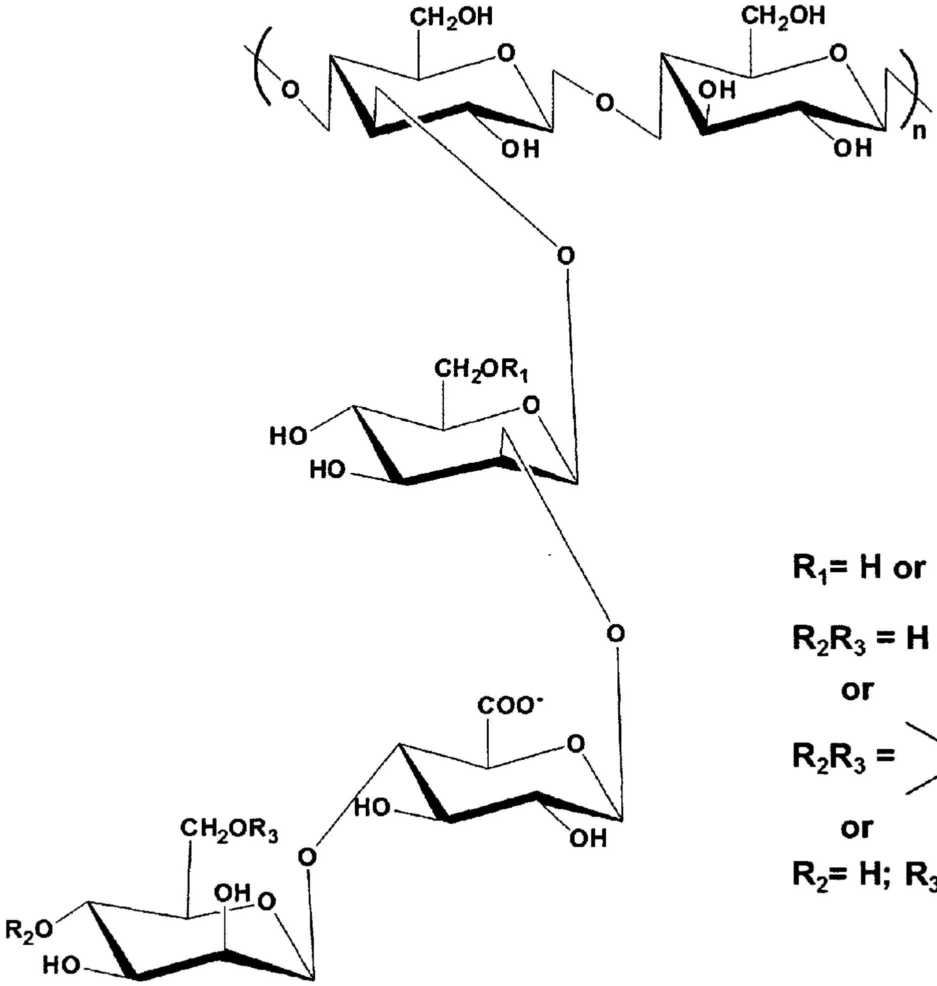

$R_1$= H or $COCH_3$

$R_2R_3$ = H

or

$R_2R_3$ = 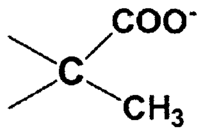

or

$R_2$= H; $R_3$ = $COCH_3$

Fig. 1B

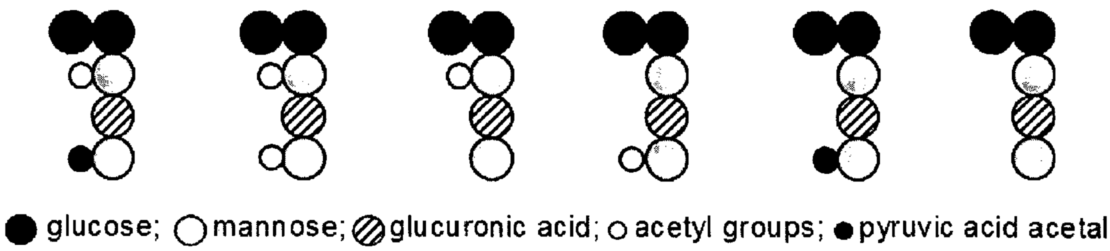

Fig. 2A

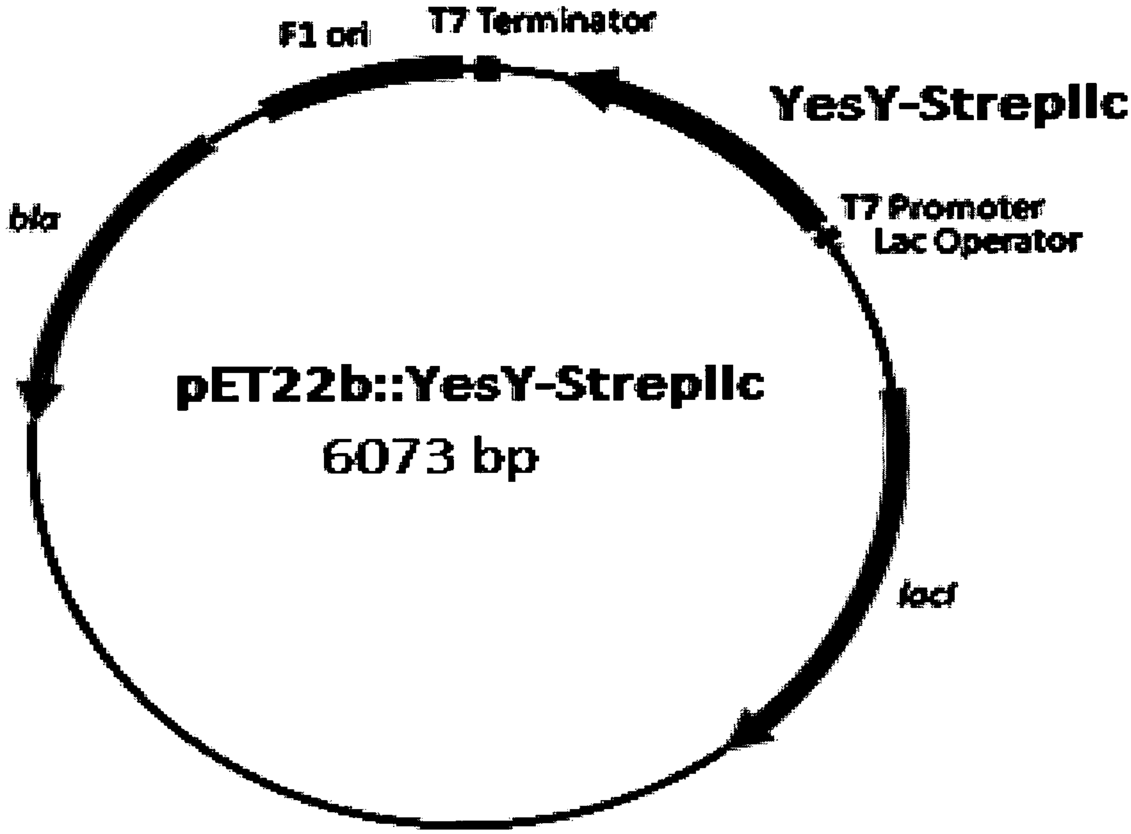
F1 ori
T7 Terminator
YesY-StrepIIc
T7 Promoter
Lac Operator
bla
pET22b::YesY-StrepIIc
6073 bp
lacI

Fig. 2B

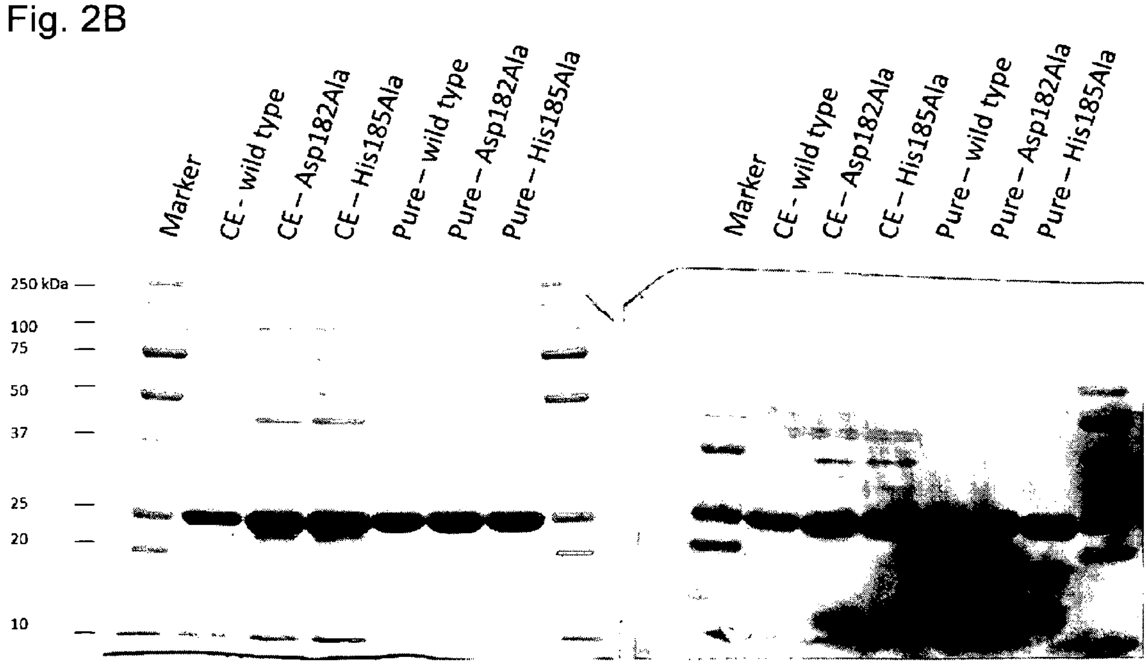
Marker
CE - wild type
CE – Asp182Ala
CE – His185Ala
Pure – wild type
Pure – Asp182Ala
Pure – His185Ala
Marker
CE - wild type
CE – Asp182Ala
CE – His185Ala
Pure – wild type
Pure – Asp182Ala
Pure – His185Ala
250 kDa
100
75
50
37
25
20
10

Fig. 3

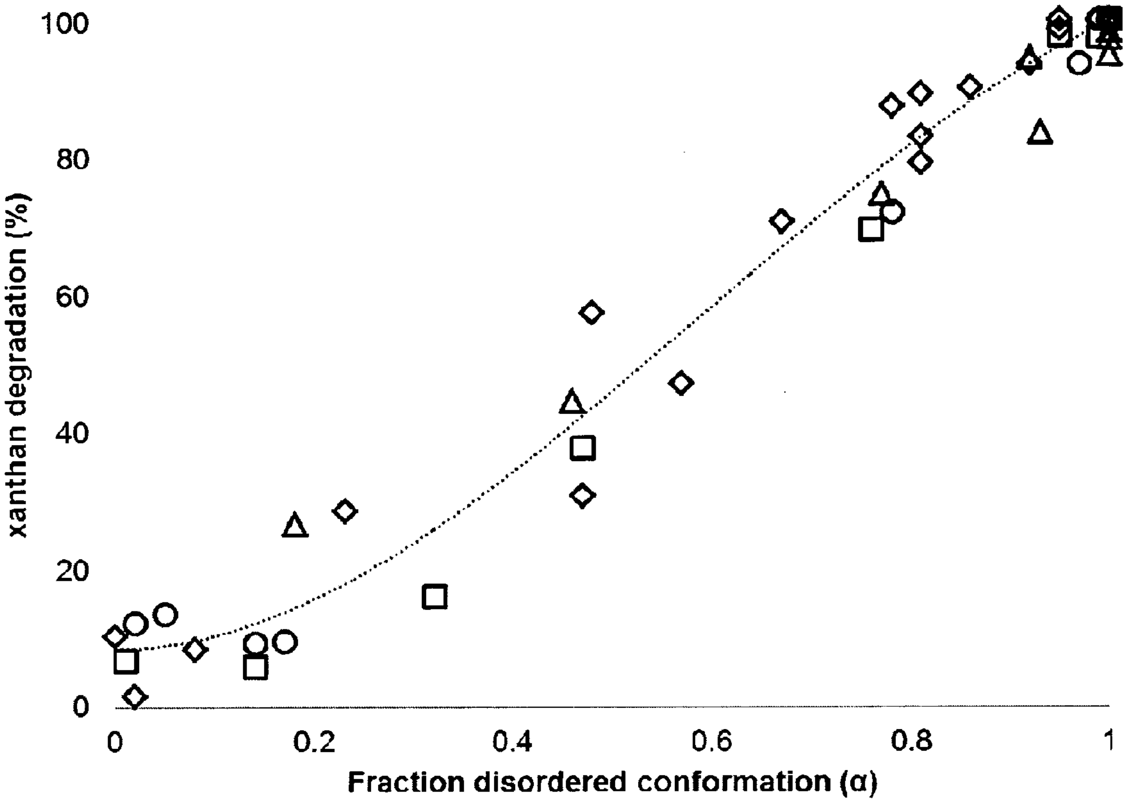
100
80
60
40
20
0
xanthan degradation (%)
0
0.2
0.4
0.6
0.8
1
Fraction disordered conformation (α)

Fig. 4

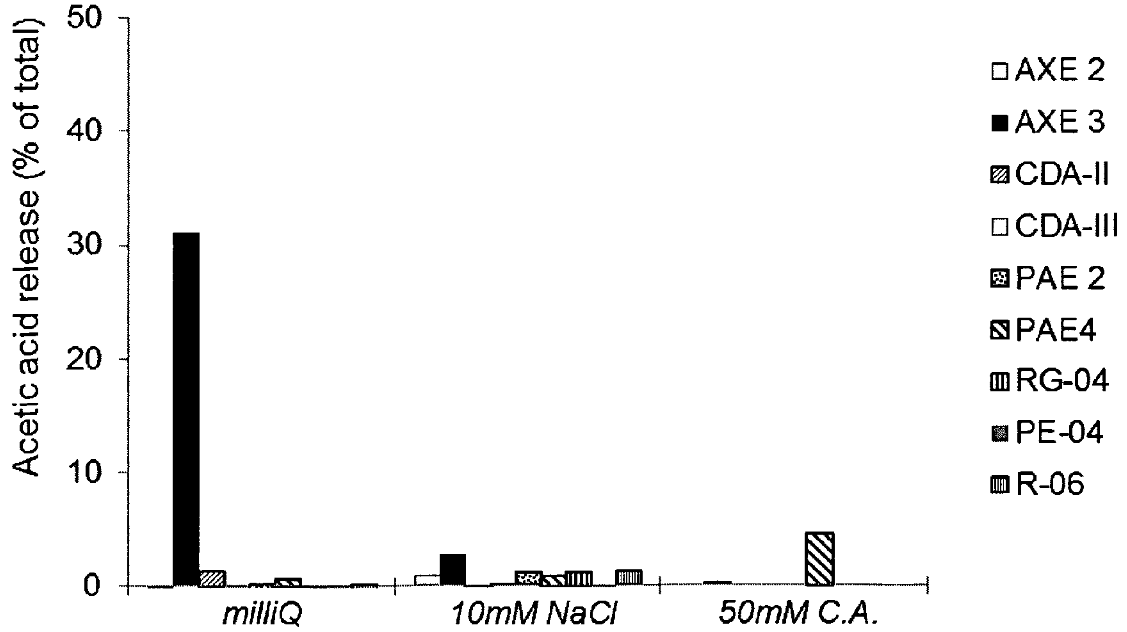
50
40
30
20
10
0
Acetic acid release (% of total)
milliQ
10mM NaCl
50mM C.A.
AXE 2
AXE 3
CDA-II
CDA-III
PAE 2
PAE4
RG-04
PE-04
R-06

Fig. 5

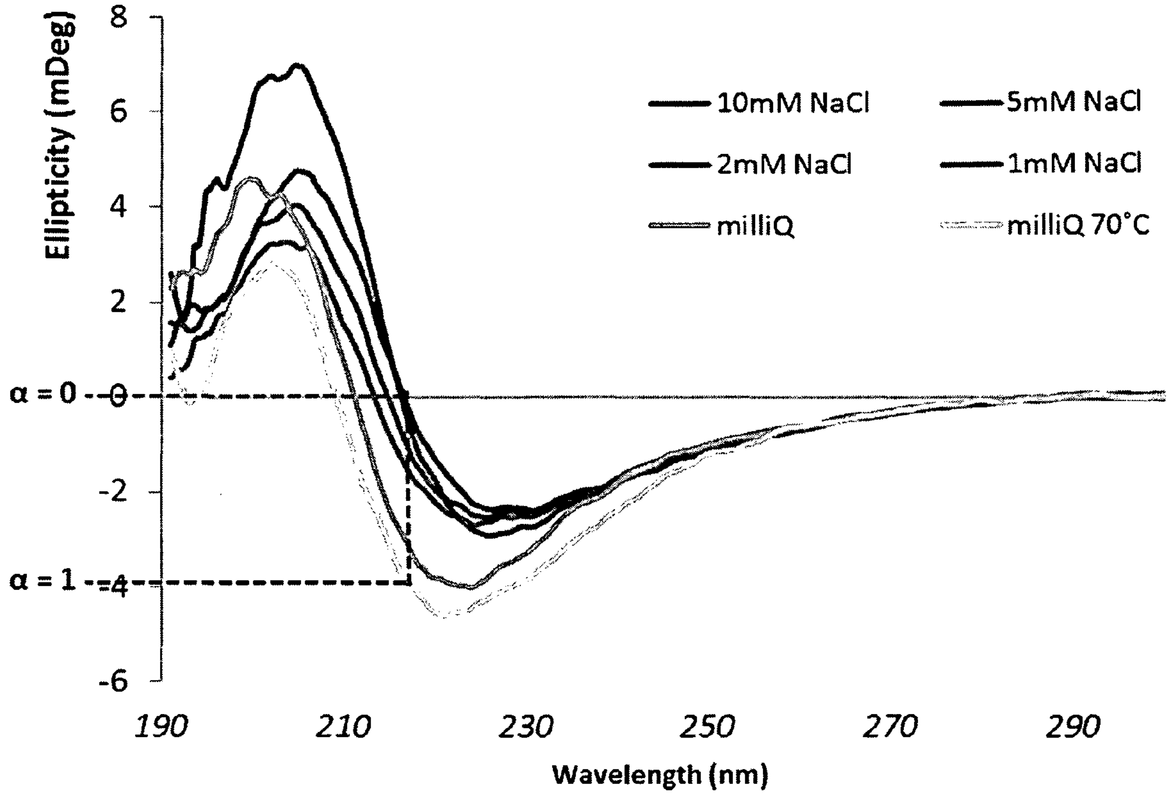
Ellipticity (mDeg)
8
6
4
2
α = 0 - - 0
-2
α = 1 - - -4
-6
190
210
230
250
270
290
Wavelength (nm)
10mM NaCl
5mM NaCl
2mM NaCl
1mM NaCl
milliQ
milliQ 70°C

Fig. 6

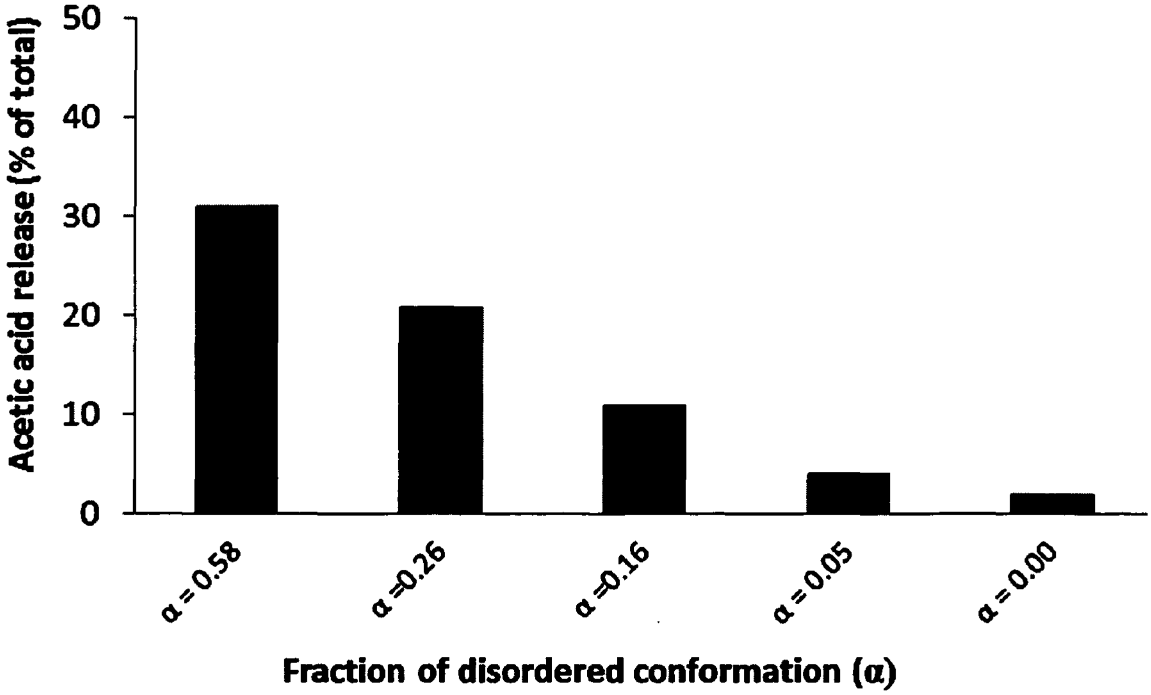
Acetic acid release (% of total)
50
40
30
20
10
0
α = 0.58
α =0.26
α =0.16
α = 0.05
α = 0.00
Fraction of disordered conformation (α)

Fig. 7

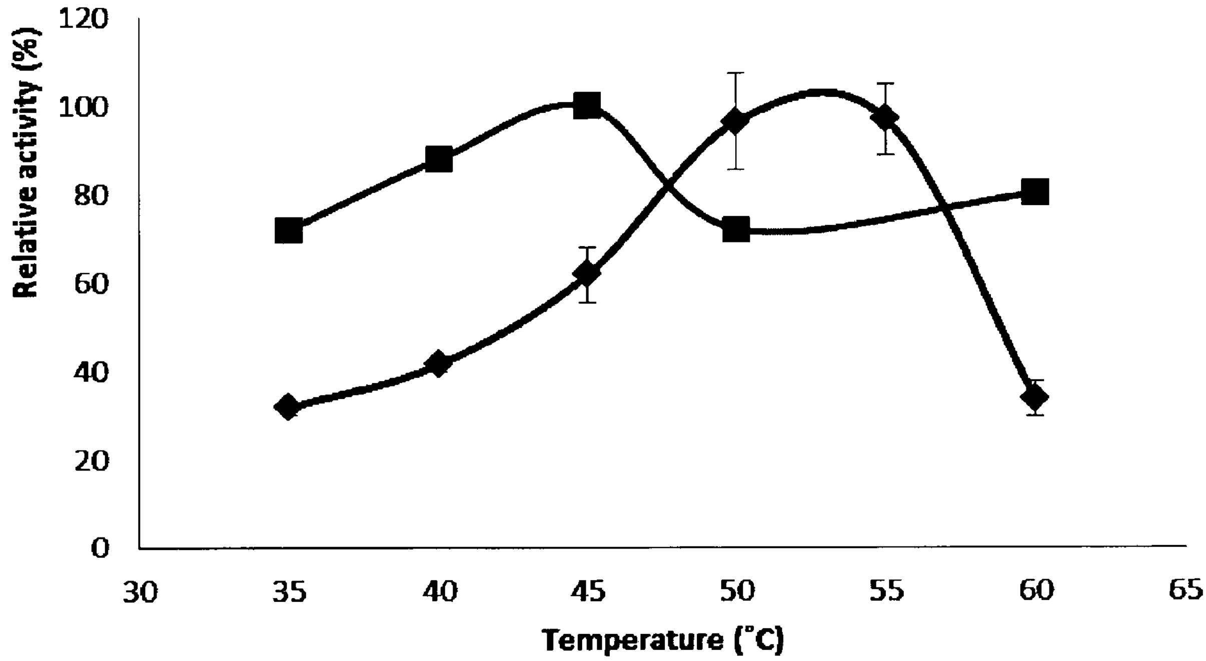
Relative activity (%)
120
100
80
60
40
20
0
30
35
40
45
50
55
60
65
Temperature (°C)

Fig. 8A

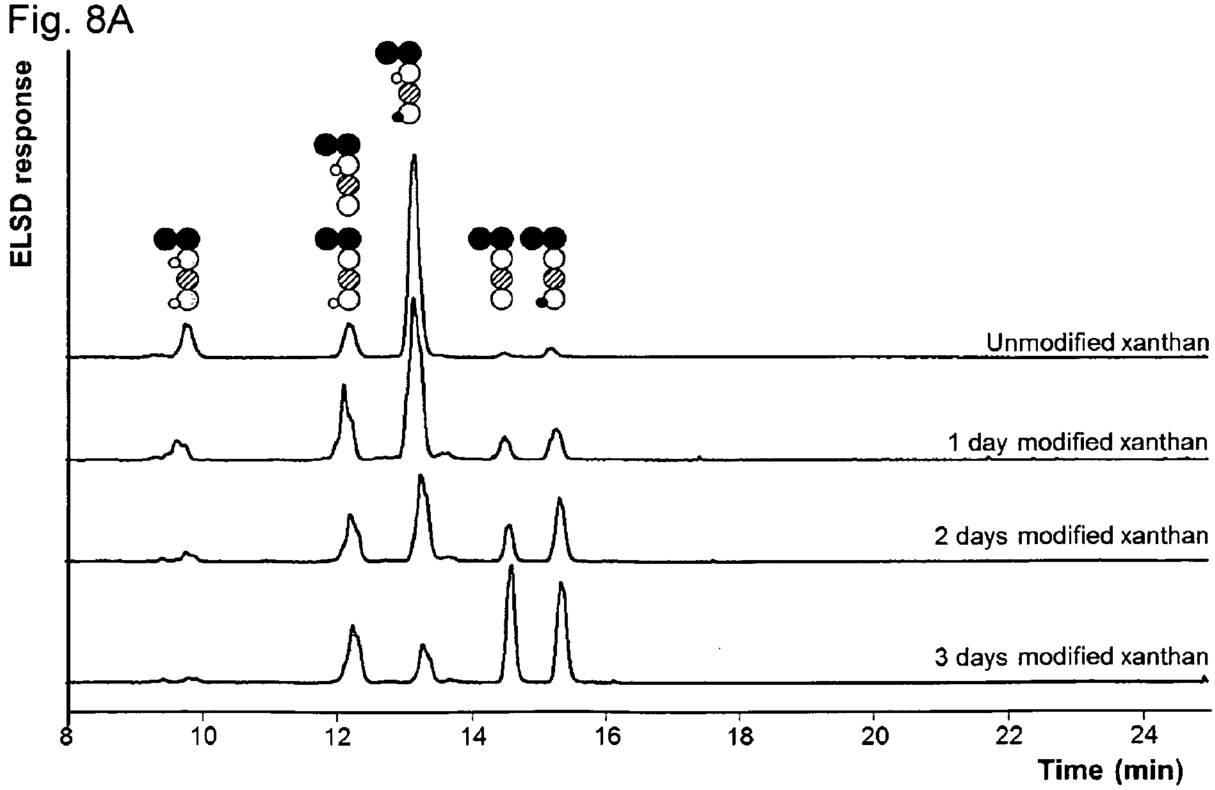
ELSD response
Unmodified xanthan
1 day modified xanthan
2 days modified xanthan
3 days modified xanthan
8
10
12
14
16
18
20
22
24
Time (min)

Fig. 8B

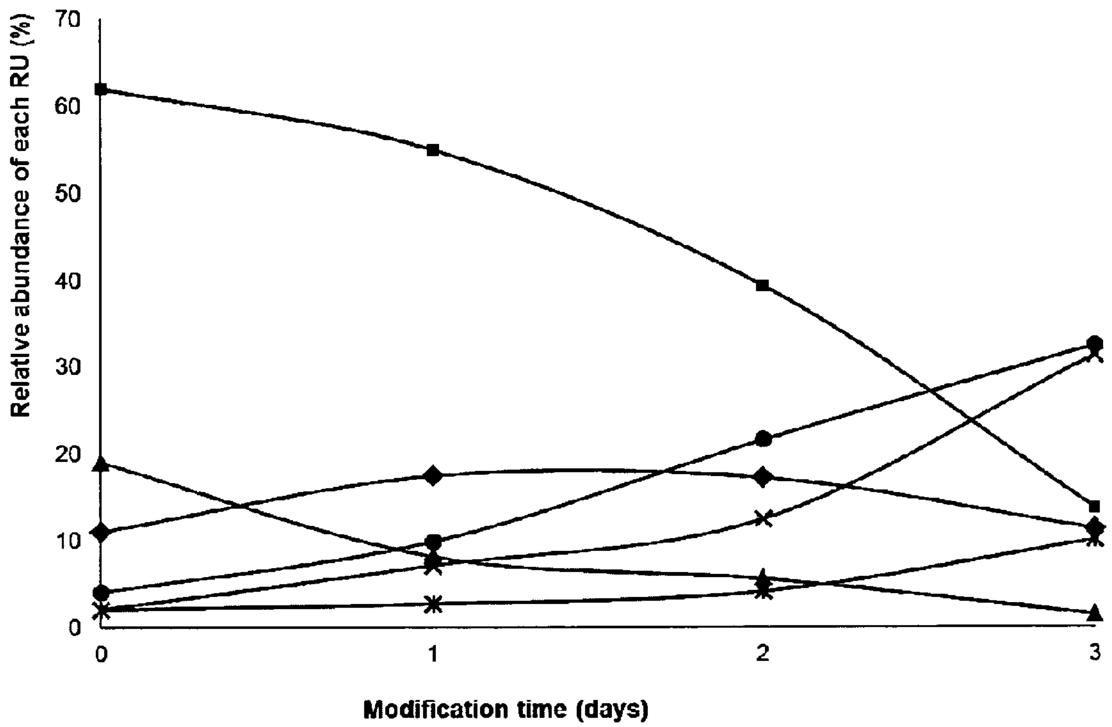
Relative abundance of each RU (%)
70
60
50
40
30
20
10
0
0
1
2
3
Modification time (days)

Fig. 9

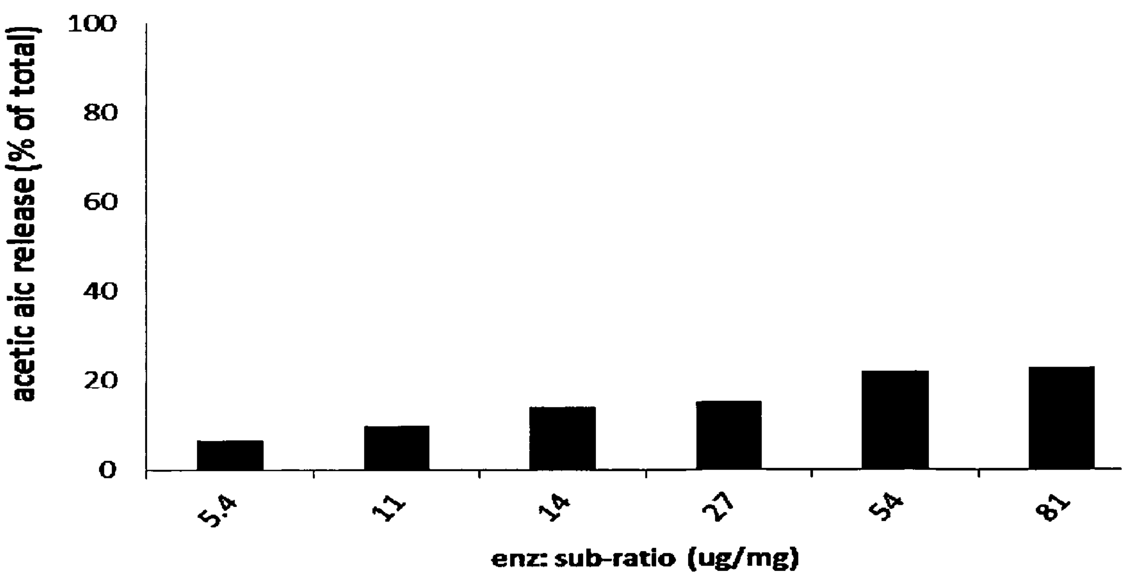
acetic aic release (% of total)
100
80
60
40
20
0
5.4
11
14
27
54
81
enz: sub-ratio (ug/mg)

Fig. 10A

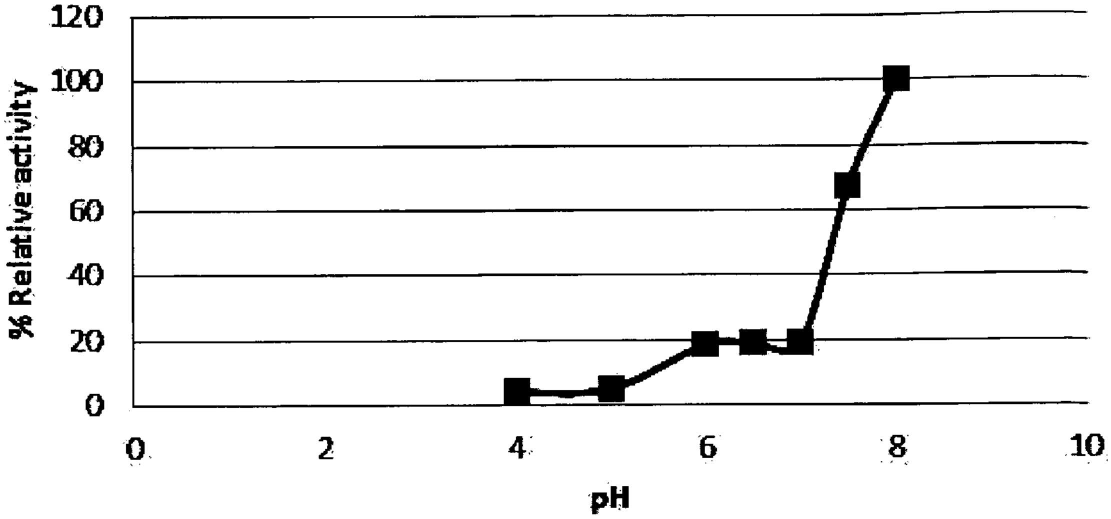


Fig. 10B

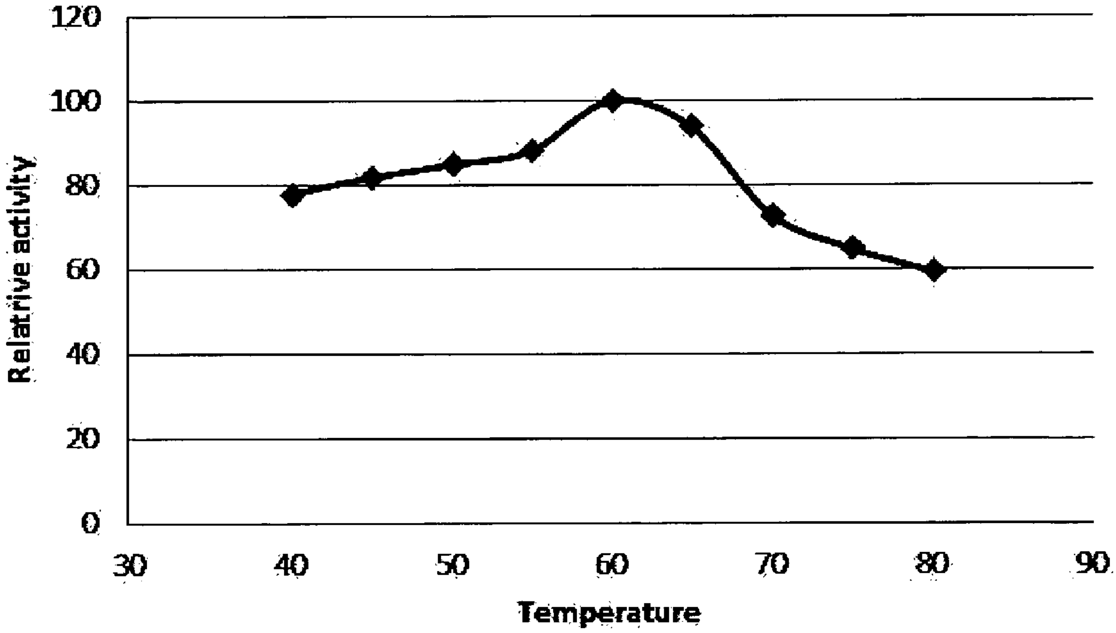

Fig. 11

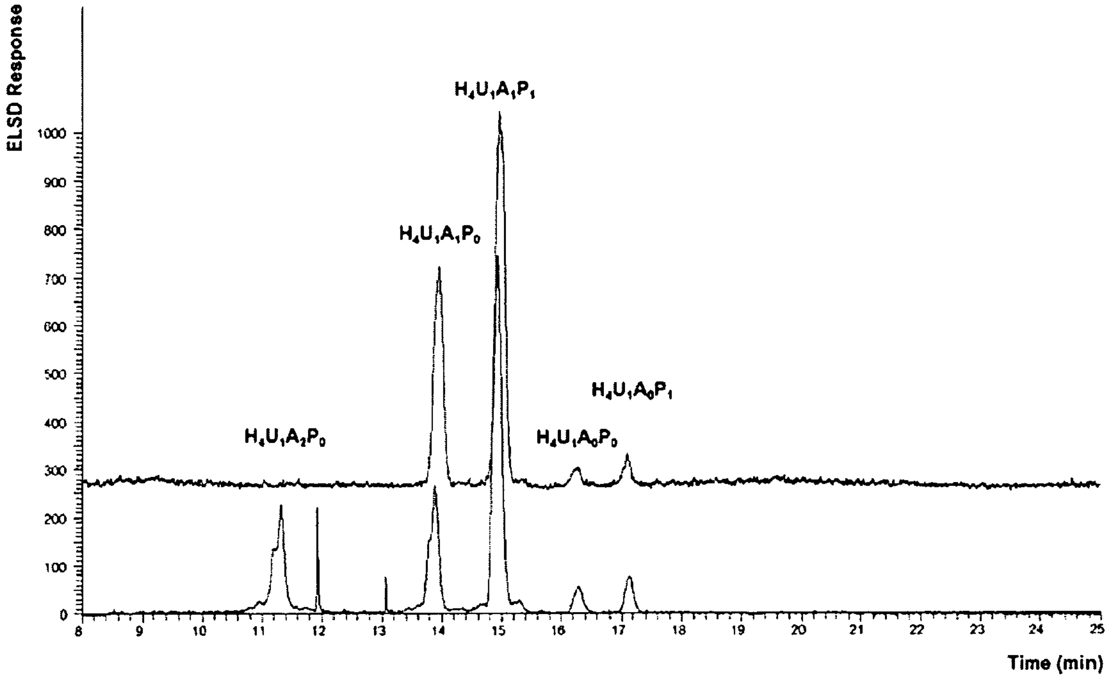
ELSD Response
1000
900
800
700
600
500
400
300
200
100
0
H4U1A1P1
H4U1A1P0
H4U1A0P1
H4U1A2P0
H4U1A0P0
8
9
10
11
12
13
14
15
16
17
18
19
20
21
22
23
24
25
Time (min)

Fig. 12

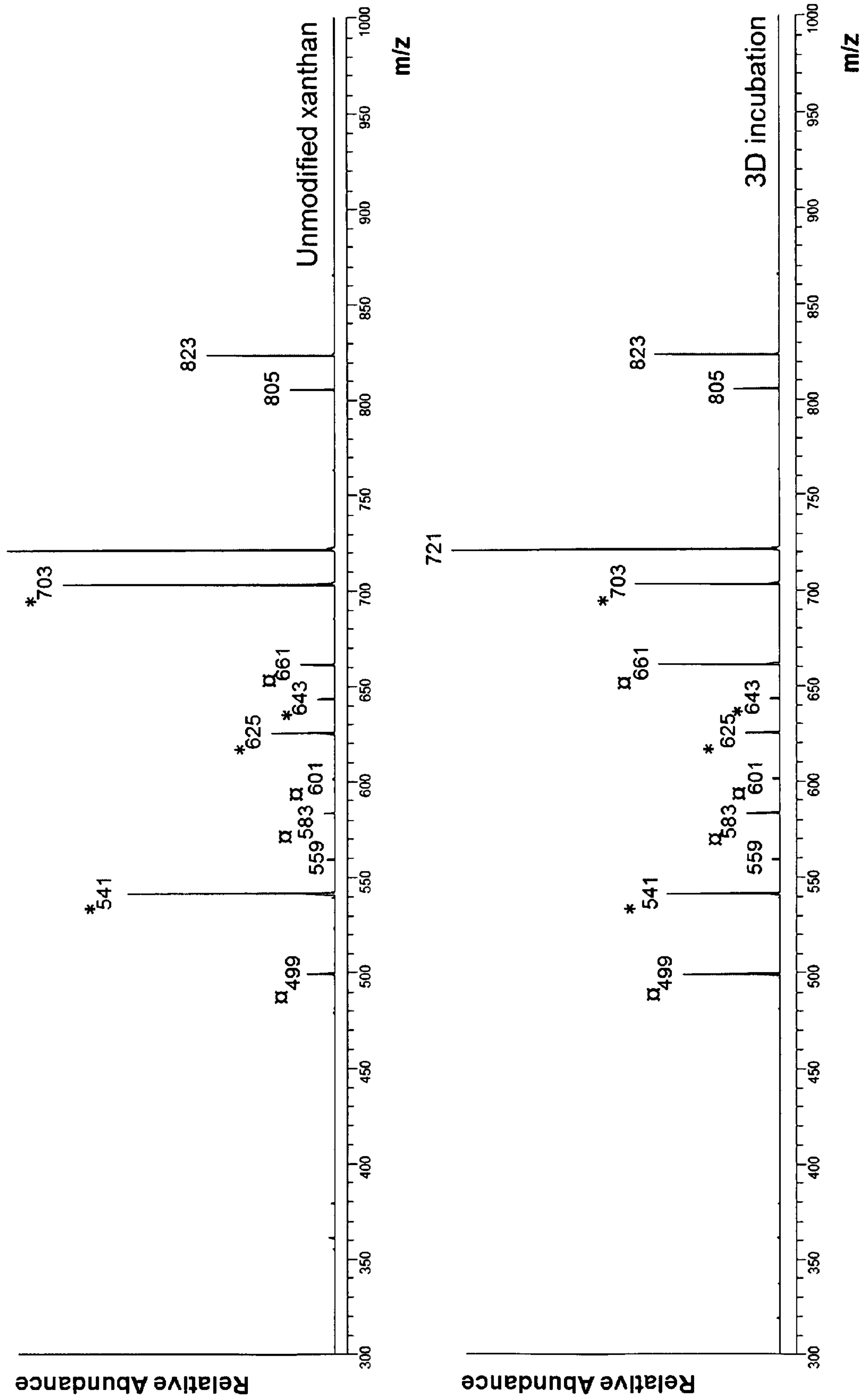

Unmodified xanthan
3D incubation
Relative Abundance
Relative Abundance
m/z
m/z
721
*703
¤661
*643
*625
¤601
¤583
559
*541
¤499
823
805
300
350
400
450
500
550
600
650
700
750
800
850
900
950
1000

Europäisches Patentamt
European Patent Office
Office européen des brevets

# EUROPEAN SEARCH REPORT

Application Number
EP 13 00 3212

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2007 041861 A1 (EVONIK DEGUSSA GMBH) 5 March 2009 (2009-03-05)<br>* page 3 - page 4, paragraph 15 *<br>* page 5, paragraph 26 - paragraph 27 *<br>* page 8; example 6 *<br>* page 9 - page 10; examples 11, 12 *<br>* page 11; example 14 *<br>* page 13; claims 1-3, 6-8, 15 *<br>----- | 1-6,15 | INV.<br>C12P19/06<br>C12N9/18<br>C08B37/00 |
| X | CUI, W. ET AL.: "Purification and characterization of an intracellular carboxylesterase from Arthrobacter viscosus NRRL B-1973",<br>ENZYME AND MICROBIAL TECHNOLOGY,<br>vol. 24, no. 3-4, 1999, pages 200-208,<br>XP002718812,<br>* abstract *<br>* page 201, column 2, line 37 - line 46 *<br>* page 202, column 2, line 27 - page 203, column 1, line 13 *<br>* page 204, column 2, line 19 - page 205, column 1, line 3 *<br>* page 205; table 3 *<br>----- | 1-6,15 | TECHNICAL FIELDS SEARCHED (IPC) |
| A | WO 92/19753 A1 (GETTY SCIENTIFIC DEVELOPMENT COMPANY)<br>12 November 1992 (1992-11-12)<br>* page 5, line 1 - line 4 *<br>* page 5, line 27 - page 6, line 14 *<br>* page 9, line 5 - line 10 *<br>* page 14, line 19 - page 15, line 11 *<br>* page 16, line 15 - line 28 *<br>* page 26 - page 28; example 4 *<br>* page 33 - page 36; examples 7, 8 *<br>* page 40; example 13 *<br>* page 45 - page 51; claims 1, 2, 4-7, 12, 13, 15-18, 36 *<br>* figures 6c-h, 10 *<br>-----<br>-/-- | 1-6,15 | C12P<br>C12N<br>C08B |

~~The present search report has been drawn up for all claims~~

2

| Place of search | Date of completion of the search | Examiner |
|---|---|---|
| Berlin | 22 January 2014 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 00 3212

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | HASSLER, R.A. & DOHERTY, D.H.: "Genetic Engineering of Polysaccharide Structure: Production of Variants of Xanthan Gum in Xanthomonas campestris", BIOTECHNOLOGY PROGRESS, vol. 6, no. 3, 1990, pages 182-187, XP001182843, * abstract * * page 183; figure 2 * * page 184, column 2, line 14 - page 185, column 1, line 4; figures 5-7 * ----- | 1-6,15 | |
| A,D | POUVREAU, L. ET AL.: "Characterization and mode of action of two acetyl xylan esterases from Chrysosporium lucknowense C1 active towards acetylated xylans", ENZYME AND MICROBIAL TECHNOLOGY, vol. 49, no. 3, 16 May 2011 (2011-05-16), pages 312-320, XP028244241, * the whole document * * see especially: * * sequence HQ324257 * * Chrysosporium lucknowense C1 acetyl xylan esterase Axe3 * ----- | 1-6,15 | TECHNICAL FIELDS SEARCHED (IPC) |
| A,D | OCHIAI, A. ET AL.: "Plant Cell Wall Degradation by Saprophytic Bacillus subtilis Strains: Gene Clusters Responsible for Rhamnogalacturonan Depolymerization", APPLIED AND ENVIRONMENTAL MICROBIOLOGY, vol. 73, no. 12, June 2007 (2007-06), pages 3803-3813, XP002718901, * the whole document * * see especially: * * ORF BSU07070 * * Bacillus subtilis strain 168 rhamnogalacturonan acetyltransferase YesY * ----- | 1-6,15 | |

~~The present search report has been drawn up for all claims~~

2

| Place of search | Date of completion of the search | Examiner |
|---|---|---|
| Berlin | 22 January 2014 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 13 00 3212

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6, 15

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 13 00 3212

The Search Division considers that the present European patent application does not comply with the requirements of unity of invention and relates to several inventions or groups of inventions, namely:

1. claims: 1-6, 15

method for producing xanthan with an altered acetylation pattern, wherein an enzyme site-specifically cleaving acetyl residues off mannose moieties of acetylated xanthan is contacted with acetylated xanthan,
use of an enzyme site-specifically cleaving off the acetyl residues off mannose moieties of acetylated xanthan

---

2. claims: 7-10

xanthan obtainable from a method of any one of claims 1 to 6

---

3. claim: 11

xanthan, wherein at least 10% of the inner mannose moieties are free of acetyl residues, optionally wherein at least 10% or at least 50% of the outer mannose moieties bear at least one acetyl residue

---

4. claim: 12

xanthan, wherein at least 10% of the outer mannose moieties are free of acetyl residues, optionally wherein at least 10% or at least 50% of the inner mannose moieties bear at least one acetyl residue

---

5. claim: 13

xanthan, wherein at least 10% of the inner and outer mannose moieties are free of acetyl residues

---

6. claim: 14

use of the xanthan according to claim 7 to 13 for modifying rheology properties, viscosity properties, thickening, gelling, preventing the formation of ice crystals, stabilizing suspensions and/or emulsions, preserving, moistening and/or hydrating skin

---

## ANNEX TO THE EUROPEAN SEARCH REPORT ON EUROPEAN PATENT APPLICATION NO. EP 13 00 3212

This annex lists the patent family members relating to the patent documents cited in the above-mentioned European search report. The members are as contained in the European Patent Office EDP file on
The European Patent Office is in no way liable for these particulars which are merely given for the purpose of information.

22-01-2014

| Patent document cited in search report | Publication date | Patent family member(s) | Publication date |
|---|---|---|---|
| DE 102007041861 A1 | 05-03-2009 | NONE | |
| WO 9219753 A1 | 12-11-1992 | AT 246255 T | 15-08-2003 |
| | | CA 2108895 A1 | 08-11-1992 |
| | | DE 69233141 D1 | 04-09-2003 |
| | | DE 69233141 T2 | 08-04-2004 |
| | | EP 0584206 A1 | 02-03-1994 |
| | | FI 934902 A | 05-11-1993 |
| | | JP H06507433 A | 25-08-1994 |
| | | NO 934044 A | 08-11-1993 |
| | | SG 48883 A1 | 18-05-1998 |
| | | WO 9219753 A1 | 12-11-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HINZ.** *Journal of cereal science,* 2009, vol. 50 (3), 318-323
- **POUVREAU.** *enzyme and microbial technology,* 2010, vol. 49 (3), 312-320
- **BEZEMER, L. ; UBBINK, J. B. ; KOOKER DE, J. A. ; KUIL, M. E. ; LEYTE, J. C.** On the conformational transitions of native xanthan. *Macromolecules,* 1993, vol. 26, 6436-6446
- **BIELY, P.** Microbial carbohydrate esterases deacetylating plant polysaccharides. *Biotechnology Advances,* 2012, vol. 30 (6), 1575-1588
- **BRADSHAW, I. J. ; NISBET, B. A. ; KERR, M. H. ; SUTHERLAND, I. W.** Modified xanthan-its preparation and viscosity. *Carbohydrate Polymers,* 1983, vol. 3 (1), 23-38
- **FLORES CANDIA, J. L. ; DECKWER, W. D.** Effect of the nitrogen source on pyruvate content and rheological properties of xanthan. *Biotechnology Progress,* 1999, vol. 15 (3), 446-452
- **HASHIMOTO, W. ; MIKI, H. ; TSUCHIYA, N. ; NANKAI, H. ; MURATA, K.** Xanthan lyase of Bacillus sp. strain GL1 liberates pyruvylated mannose from xanthan side chains. *Applied and Environmental Microbiology,* 1998, vol. 64 (10), 3765-3768
- **HASSLER, R. A. ; DOHERTY, D. H.** Genetic engineering of polysaccharide structure: Production of variants of xanthan gum in Xanthomonas campestris. *Biotechnology Progress,* 1990, vol. 6 (3), 182-187
- **HINZ, S. W. A. ; POUVREAU, L. ; JOOSTEN, R. ; BARTELS, J. ; JONATHAN, M. C. ; WERY, J. ; SCHOLS, H. A.** Hemicellulase production in Chrysosporium lucknowense C1. *Journal of Cereal Science,* 2009, vol. 50 (3), 318-323
- **JANSSON, P. E. ; KENNE, L. ; LINDBERG, B.** Structure of extracellular polysaccharide from Xanthomonas campestris. *Carbohydrate Research,* December 1975, vol. 45, 275-282
- **KOOL, M. M. ; SCHOLS, H. A. ; DELAHAIJE, R. J. B. M. ; SWORN, G. ; WIERENGA, P. A. ; GRUPPEN, H.** The influence of the primary and secondary xanthan structure on the enzymatic hydrolysis of the xanthan backbone. *Carbohydrate Polymers,* 2013, vol. 97 (2), 368-375
- **KOOL, M.M. ; GRUPPEN, H. ; SWORN, G. ; SCHOLS, H.A.** Comparison of xanthans by the relative abundance of its six constituent repeating units. Accepted for publication. *Carbohydrate Polymers, minor revisions,* June 2013
- **KORMELINK, F. J. M. ; LEFEBVRE, B. ; STROZYK, F. ; VORAGEN, A. G. J.** Purification and characterization of an acetyl xylan esterase from Aspergillus niger. *Journal of Biotechnology,* 1993, vol. 27 (3), 267-282
- **MATSUDA, Y. ; BIYAJIMA, Y. ; SATO, T.** Thermal denaturation, renaturation, and aggregation of a double-helical polysaccharide xanthan in aqueous solution. *Polymer Journal,* 2009, vol. 41 (7), 526-532
- **MORRIS, E. R. ; REES, D. A. ; YOUNG, G.** Order disorder transition for a bacterial polysaccharide in solution. A role for polysaccharide conformation in recognition between Xanthomonas pathogen and its plant host. *Journal of Molecular Biology,* 1977, vol. 110 (1), 1-16
- New forms of xanthan gum with enhanced properties. **MORRISON, N. A. ; CLARK, R. ; TALASHEK, T. ; YUAN, C. R.** Gums and Stabilisers for Food Industry. RSC, 2004, vol. 12, 124-130
- **PELLETIER, E. ; VIEBKE, C. ; MEADOWS, J. ; WILLIAMS, P. A.** A rheological study of the order-disorder conformational transition of xanthan gum. *Biopolymers,* 2001, vol. 59, 339-346
- **OCHIAI A ; TAKAFUMI I ; KAWAMATA A ; WATARU H ; MURATA K.** Plant cell wall degradation by saprophytic Bacillus subtilis strains: gene clusters responsible for rhamnogalacturonan depolymerization. *Appl. Environ. Microbiol,* 2007, vol. 73 (12), 3803-3813
- **PETERS, H. U. ; SUH, I. S. ; SCHUMPE, A. ; DECKWER, W. D.** The pyruvate content of xanthan polysaccharide produced under oxygen limitation. *Biotechnology Letters,* 1993, vol. 15 (6), 565-566
- **PINTO, E. P. ; FURLAN, L. ; VENDRUSCOLO, C. T.** Chemical deacetylation natural xanthan (Jungbunzlauer®. *Polimeros,* 2011, vol. 21 (1), 47-52
- **POUVREAU, L. ; JONATHAN, M. C. ; KABEL, M. A. ; HINZ, S. W. A. ; GRUPPEN, H. ; SCHOLS, H. A.** Characterization and mode of action of two acetyl xylan esterases from Chrysosporium lucknowense C1 active towards acetylated xylans. *Enzyme and Microbial Technology,* 2011, vol. 49 (3), 312-320
- **RINAUDO, M. ; MILAS, M.** Enzymic-hydrolysis of the bacterial polysaccharide xanthan by cellulase. *International Journal of Biological Macromolecules,* 1980, vol. 2 (1), 45-48

- **RUIJSSENAARS, H. J. ; DE BONT, J. A. M. ; HARTMANS, S.** A pyruvated mannose-specific xanthan lyase involved in xanthan degradation by Paenibacillus alginolyticus XL-1. *Applied and Environmental Microbiology,* 1999, vol. 65 (6), 2446-2452
- **SANDFORD, P. A. ; PITTSLEY, J. E. ; KNUTSON, C. A. ; CADMUS, M. C. ; WATSON, P. R. ; JEANES, A.** Variation in Xanthomonas campestris NRRL B-1459; Characterisation of xanthan samples of different pyruvic acid content. *Extracellular Microbial Polysaccharides,* 1977, 192-210
- **SEARLE-VAN LEEUWEN, M. J. F. ; BROEK, L. A. M. ; SCHOLS, H. A. ; BELDMAN, G. ; VORAGEN, A. G. J.** *Rhamnogalacturonan acetylesterase: a novel enzyme from Aspergillus aculeatus, specific for the deacetylation of hairy (ramified) regions of pectins,* 1992, vol. 38 (3), 347-349
- Acetyl esterases of Aspergillus niger: purification and mode of action on pectins. **SEARLE-VAN LEEUWEN, M. J. F. ; VINCKEN, J. P. ; SCHIPPER, D. ; VORAGEN, A. G. J. ; BELDMAN, G. ; VISSER, J. ; VORAGEN, A. G. J.** Progress in Biotechnology. Elsevier, 1996, 793-798
- **SHATWELL, K. P. ; SUTHERLAND, I. W. ; DEA, I. C. M. ; ROSS-MURPHY, S. B.** The influence of acetyl and pyruvate substituents on the helix-coil transition behaviour of xanthan. *Carbohydrate Research,* 1990, vol. 206 (1), 87-103
- **SHATWELL, K. P. ; SUTHERLAND, I. W. ; ROSS-MURPHY, S. B. ; DEA, I. C. M.** Influence of the acetyl substituent on the interaction of xanthan with plant polysaccharides - I. Xanthan-locust bean gum systems. *Carbohydrate Polymers,* 1990, vol. 14 (1), 29-51
- **SHATWELL, K. P. ; SUTHERLAND, I. W. ; ROSS-MURPHY, S. B. ; DEA, I. C. M.** Influence of the acetyl substituent on the interaction of xanthan with plant polysaccharides - II. Xanthan-guar gum systems. *Carbohydrate Polymers,* 1991, vol. 14 (2), 115-130
- **SMITH, C. J. H. ; SYMES, K. C. ; LAWSON, C. J. ; MORRIS, E. R.** The effect of pyruvate on xanthan solution properties. *Carbohydrate Polymers,* 1984, vol. 4, 153-157
- **STANKOWSKI, J. D. ; MUELLER, B. E. ; ZELLER, S. G.** Location of a second O-acetyl group in xanthan gum by reductive-cleavage method. *Carbohydrate Research,* 1993, vol. 241 (1), 321-326
- **TAKO, M. ; NAKAMURA, S.** Rheology properties of deacetylated xanthan in aqueous-media. *Agricultural and Biological Chemistry,* 1984, vol. 48 (12), 2987-2993
- **TOPAKAS, E. ; KYRIAKOPOULOS, S. ; BIELY, P. ; HIRSCH, J. ; VAFIADI, C. ; CHRISTAKOPOULOS, P.** Carbohydrate esterases of family 2 are 6-O-deacetylases. *FEBS Letters,* 2010, vol. 584 (3), 543-548
- **VISSER, H. ; JOOSTEN, V. ; PUNT, P. J. ; GUSAKOV, A. V. ; OLSON, P. T. ; JOOSTEN, R. ; BARTELS, J. ; VISSER, J. ; SINITSYN, A. P. ; EMALFARB, M. A.** Development of a mature fungal technology and production platform for industrial enzymes based on a Myceliophthora thermophila isolate, previously known as Chrysosporium lucknowense C1. *Industrial Biotechnology,* 2011, vol. 7 (3), 214-223